Europäisches Patentamt
European Patent Office
Office européen des brevets

(11) Veröffentlichungsnummer: **0 228 343**
**A2**

# (12) EUROPÄISCHE PATENTANMELDUNG

(21) Anmeldenummer: **86810524.8**

(22) Anmeldetag: **17.11.86**

(51) Int. Cl.[4]: **C 07 D 249/08**
C 07 D 405/06,
A 01 N 43/653, C 07 D 317/16

(30) Priorität: **22.11.85 CH 4985/85**

(43) Veröffentlichungstag der Anmeldung:
**08.07.87 Patentblatt 87/28**

(84) Benannte Vertragsstaaten:
**BE CH DE ES FR GB GR IT LI LU NL**

(71) Anmelder: **CIBA-GEIGY AG**
**Klybeckstrasse 141**
**CH-4002 Basel (CH)**

(72) Erfinder: **Hubele, Adolf, Dr.**
**Obere Egg 9**
**CH-4312 Magden (CH)**

Die Bezeichnung der Erfindung wurde geändert (Richtlinien für die Prüfung im EPA, A-III, 7.3).

(54) **Mikrobizide 2-(1-Triazolyl)1-phenyl-ethanon-(1)-Ketalderivate.**

(57) Neue substituierte 2-(1H-1,2,4-Triazolyl)-1-phenyl-äthanon(1)-ketale der allgemeinen Formel

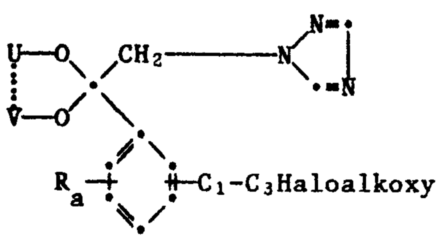

in welcher einer der beiden Phenylsubstituenten in 2-Stellung und der andere in 4-Stellung steht, und worin
$R_a$ Halogen, Methyl oder $C_1$-$C_3$-Haloalkoxy bedeutet, und
U und V unabhängig voneinander für gegebenenfalls durch Halogen oder $C_1$-$C_6$-Alkoxy substituiertes $C_1$-$C_{12}$-Alkyl darstellen oder zusammen eine der folgenden Alkylenbrücken

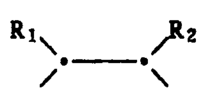

oder

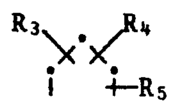

bilden, wobei

$R_1$ bis $R_5$ die hierin definierten Substituenten darstellen, sowie ihre Säureadditionssalze und Metallsalzkomplexe besitzen mikrobizide Eigenschaften und eignen sich insbesondere zur Bekämpfung phytopathogener Mikroorganismen. Sie lassen sich in Form von Pflanzenschutz- oder Saatbeiz-Mitteln zur Verhütung oder Bekämpfung von Pflanzenkrankheiten einsetzen.

EP 0 228 343 A2

**Beschreibung**

Mikrobizide Wirkstoffe

Die vorliegende Erfindung betrifft neue, durch Haloalkoxy substituierte 2-(1H-1,2,4-Triazolyl)-1-phenyl-äthanon(1)-ketale der nachstehenden Formel I, einschliesslich ihrer Säureadditionssalze und Metallkomplexe. Die Erfindung betrifft ferner die Herstellung dieser Verbindungen sowie mikrobizide Mittel, die als mindestens eine Wirkstoffkomponente eine der Verbindungen der Formel I enthalten. Sie betrifft weiterhin die Herstellung der genannten Mittel und die Verwendung der Wirkstoffe bzw. der Mittel zur Bekämpfung von schädlichen Mikroorganismen.

Die erfindungsgemässen Verbindungen haben die Formel I

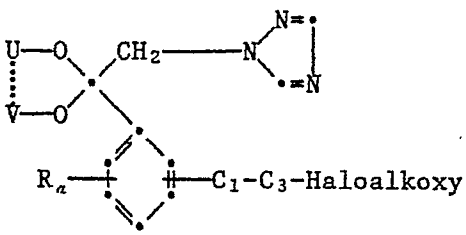

(I)

in welcher einer der beiden Phenylsubstituenten in 2-Stellung und der andere in 4-Stellung steht, und worin $R_a$ Halogen, Methyl oder $C_1$-$C_3$-Haloalkoxy bedeutet, und worin
U und V unabhängig voneinander gegebenenfalls durch Halogen oder $C_1$-$C_6$-Alkoxy substituiertes $C_1$-$C_{12}$-Alkyl darstellen oder zusammen eine der folgenden Alkylenbrücken

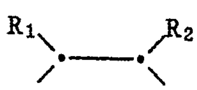 oder 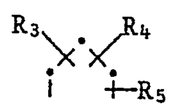 bilden, wobei

$R_1$ und $R_2$ unabhängig voneinander für Wasserstoff, $C_1$-$C_6$-Alkyl, ein- oder mehrfach durch Halogen substituiertes $C_1$-$C_6$-Alkyl, Phenyl, ein- oder mehrfach durch Halogen und/oder $C_1$-$C_2$-Alkyl substituiertes Phenyl oder die Gruppe $-CH_2$-Z-$R_6$ stehen oder worin $R_1$ und $R_2$ zusammen eine unsubstituierte oder durch $C_1$-$C_4$-Alkyl substituierte Tetramethylenbrücke bilden; und wobei
Z Sauerstoff oder Schwefel bedeutet;
$R_6$ für Wasserstoff, $C_1$-$C_6$-Alkyl, ein- oder mehrfach durch Halogen oder $C_1$-$C_3$-Alkoxy substituiertes $C_1$-$C_6$-Alkyl, oder für $C_3$-$C_4$-Alkenyl, 2-Propinyl (=Propargyl), 3-Halo-2-propinyl, Phenyl, oder Benzyl, deren aromatischer Ring unsubstituiert oder durch Halogen, $C_1$-$C_3$-Alkyl, $C_1$-$C_3$-Alkoxy, Nitro und/oder $CF_3$ ein-oder mehrfach substituiert ist, steht;
$R_3$, $R_4$ und $R_5$ unabhängig voneinander Wasserstoff oder $C_1$-$C_4$-Alkyl bedeuten, wobei die Gesamtzahl der Kohlenstoffatome in $R_3$, $R_4$ und $R_5$ die Zahl 6 nicht übersteigt;
unter Einschluss ihrer Säureadditionssalze und Metallsalzkomplexe.

Unter dem Begriff Alkyl selbst oder als Bestandteil eines anderen Substituenten sind je nach Zahl der angegebenen Kohlenstoffatome beispielsweise folgende Gruppen zu verstehen: Methyl, Ethyl, Propyl, Butyl, Pentyl, Hexyl, Heptyl, Octyl, Nonyl, Decyl, Undecyl oder Dodecyl sowie ihre Isomeren wie z.B. Isopropyl, Isobutyl, tert.-Butyl, sek.-Butyl, Isopentyl. Alkenyl steht z.B. für Propenyl-(1), Allyl, Butenyl-(1), Butenyl-(2) oder Butenyl-(3). Halogen oder Halo bzw. Hal soll hier und im folgenden Fluor, Chlor, Brom oder Jod, vorzugsweise Fluor, Chlor oder Brom, bedeuten.

Haloalkoxy steht je nach Zahl der Kohlenstoffatome für eine geradkettige einfach- bis perhalogenierte, über Sauerstoff gebundene Alkylgruppe, die gleiche oder unterschiedliche Halogenatome aufweist. Beispiele derartiger halogenierter Gruppen sind: $-OCH_2F$, $-OCHF_2$, $-OCF_3$, $-OCH_2Cl$, $-OCCl_3$, $-OCHFCl$, $-OCClF_2$, $-OCF_2CHF_2$, $-OCF_2CFCl_2$, $-OCF_2CHFCl$, $-OCF_2CF_2Cl$, $-OCF_2CCl_3$, $-OCF_2CHCl_2$, $-OCF_2CH_3$, $-OCH_2CF_3$, $OCH_2CH_2Cl$, $-OCH_2CH_2F$, $-OCH_2CF_2Br$, $-OCClFCFCl_2$, $-OCHFCF_2Br$, oder $-OCF_2CHFCF_3$.

Die Erfindung betrifft sowohl die freien Verbindungen der Formel I als auch deren Additionssalze mit anorganischen und organischen Säuren sowie deren Komplexe mit Metallsalzen.

Erfindungsgemässe Salze sind insbesondere Additionssalze mit nach Massgabe des Einsatzzweckes physiologisch unbedenklichen anorganischen oder organischen Säuren.

Bezüglich des Einsatzes als Mikrobizide im Pflanzenschutz physiologisch geeignete anorganische und organische Säuren sind beispielsweise Halogenwasserstoffsäuren, z.B. Chlor-, Brom- oder Jodwasserstoffsäure, Schwefelsäure, Phosphorsäure, phosphorige Säure, Salpetersäure, gegebenenfalls halogenierte Fettsäuren, wie Essigsäure oder Trichloressigsäure, Oxalsäure, oder Sulfonsäuren, wie Benzolsulfonsäure und Methansulfonsäure.

Metallsalzkomplexe der Formel I bestehen aus dem zugrundeliegenden organischen Molekül und einem

anorganischen oder organischen Metallsalz, z.B. den Halogeniden, Nitraten, Sulfaten, Phosphaten, Tartraten usw. des Kupfers, Mangans, Eisens, Zinks und anderer Metalle. Dabei können die Metallkationen in den verschiedenen ihnen zukommenden Wertigkeiten vorliegen.

Die Verbindungen der Formel I sind bei Raumtemperatur stabile Oele, Harze oder Feststoffe, die sich durch sehr wertvolle physiologische, wie mikrobizide, insbesondere pflanzenfungizide Eigenschaften auszeichnen. Sie lassen sich daher sehr wirkungsvoll auf dem Agrarsektor oder verwandten Gebieten zur Bekämpfung von phytopathogenen Mikroorganismen einsetzen. Dabei weisen die erfindungsgemässen, im Phenylkern haloalkoxylierten Verbindungen im Vergleich zu den struckturnächsten Verbindungen des Standes der Technik eine signifikant verbesserte mikrobizide Wirkung und eine günstige biologische Abbaubarkeit auf, so dass ihre Anwendung in vorteilhafter Weise zur Entlastung der Umwelt beiträgt.

Aufgrund ihrer ausgeprägten mikrobiziden Wirkung sind diejenigen Wirksubstanzen der Formel I bevorzugt, die folgende Substituenten oder Kombinationen dieser Substituenten untereinander aufweisen.

Es sind Verbindungen der Formel I bevorzugt, worin der Substituent "$C_1$-$C_3$ Haloalkoxy" mindestens eines oder aber mehrere gleiche oder verschiedene Halogenatome ausgewählt aus Fluor, Chlor und Brom enthält und 0-4 Wasserstoffatome besitzt, abhängig von den durch die Zahl der C-Atome gegebenen Möglichkeiten; $R_a$ für Fluor, Chlor, Brom, Methyl oder für ein beliebiges, wie vorstehend definiertes $C_1$-$C_3$ Haloalkoxy steht; U und V unabhängig voneinander $C_1$-$C_6$ Alkyl darstellen oder zusammen eine der für Formel I genannten Alkylenbrücken bilden, worin $R_1$ Wasserstoff oder $C_1$-$C_2$Alkyl, und
$R_2$ $C_1$-$C_6$ Alkyl, ein- oder mehrfach durch Halogen substituiertes $C_1$-$C_6$ Alkyl, Phenyl, ein- bis dreifach durch Halogen und/oder $C_1$-$C_2$ Alkyl substituiertes Phenyl oder die Gruppe -$CH_2$-O-$R_6$ stehen, bei der
$R_6$ Wasserstoff, $C_1$-$C_6$ Alkyl, ein- oder mehrfach durch Halogen oder $C_1$-$C_3$ Alkoxy substituiertes $C_1$-$C_3$ Alkyl oder aber $C_3$-$C_4$ Alkenyl oder Propargyl darstellt, Phenyl oder Benzyl bedeutet, deren aromatischer Ring unsubstituiert oder durch Halogen, $C_1$-$C_2$ Alkyl, $C_1$-$C_2$ Alkoxy, Nitro und/oder $CF_3$ ein- bis dreifach substituiert ist; und worin $R_1$ und $R_2$ zusammen auch eine unsubstituierte oder methylsubstituierte Tetramethylenbrücke bilden können, und worin $R_3$, $R_4$ und $R_5$ unabhängig voneinander Wasserstoff oder Alkylgruppen mit insgesamt maximal 4 Kohlenstoffatomen bedeuten (Verbindungsgruppe Ia).

Innerhalb der vorgenannten Verbindungsgruppe Ia sind solche Verbindungen der Formel I bevorzugt, bei denen die $C_1$-$C_3$ Haloalkoxygruppe eine der folgenden Bedeutungen hat:

| | | |
|---|---|---|
| A) $-OCHF_2$ | H) $-OCF_2-CHFBr$ | O) $-OCBr_3$ |
| B) $-OCF_2-CHF_2$ | I) $-OCH_2-CF_3$ | P) $-OCF_2Br$ |
| C) $-OCF_2-CFCl_2$ | J) $-OCH_2-CH_2Cl$ | Q) $-OC_2F_5$ |
| D) $-OCF_2-CHCl_2$ | K) $-OCH_2-CH_2F$ | R) $-OCF_3$ |
| E) $-OCF_2-CHFCl$ | L) $-OCH_2-CCl_3$ | S) $-OCH_2Cl$ |
| F) $-OCF_2-CCl_3$ | M) $-OCF_2-CHF-CF_3$ | T) $-OCHFCl$ |
| G) $-OCF_2-CFCl_2$ | N) $-OCCl_3$ | U) $-OCH_2Br$; |

$R_a$ für Fluor, Chlor, Brom, Methyl oder für eine beliebige $C_1$-$C_3$ Haloalkoxygruppe vorstehender Bedeutung A) bis U) steht;
U und V unabhängig voneinander $C_1$-$C_6$ Alkyl darstellen oder zusammen eine der für Formel I genannten Alkylenbrücken bilden, worin $R_1$ Wasserstoff oder $C_1$-$C_2$ Alkyl und
$R_2$ $C_1$-$C_6$ Alkyl, ein- oder mehrfach durch Fluor oder Chlor substituiertes $C_1$-$C_3$ Alkyl, Phenyl, ein- oder zweifach durch Halogen und/oder Methyl substituiertes Phenyl oder die Gruppe -$CH_2$-O-$R_6$ bedeuten, bei der $R_6$ $C_1$-$C_6$ Alkyl, ein- oder mehrfach durch Fluor, und/oder Chlor substituiertes oder aber durch $C_1$-$C_3$-Alkoxy substituiertes $C_1$-$C_3$ Alkyl bedeutet oder aber $C_3$-$C_4$ Alkenyl, Propargyl oder Phenyl oder Benzyl ist, deren aromatischer Ring unsubstituiert oder durch Fluor, Chlor, Brom, Methyl, Methoxy, Nitro und/oder $CF_3$ ein- oder zweifach substituiert ist, und worin $R_1$ und $R_2$ zusammen auch eine unsubstituierte oder methylsubstituierte Tetramethylenbrücke bilden können; und worin $R_3$ Wasserstoff und $R_4$ und $R_5$ unabhängig voneinander Wasserstoff, Methyl, Aethyl oder n-Propyl bedeuten, zusammen jedoch 0 bis 4 Kohlenstoffatome besitzen (Verbindungsgruppe Ib).

Innerhalb der letztgenannten Verbindungsgruppe Ib sind solche Verbindungen der Formel I von Bedeutung, bei denen die $C_1$-$C_3$ Haloalkoxygruppe eine der folgenden Bedeutungen hat:
A) $-OCHF_2$ F) $-OCF_2-CCl_3$
B) $-OCF_2-CHF_2$ G) $-OCF_2-CFCl_2$
C) $-OCF_2-CFCl_2$ H) $-OCF_2-CHFBr$
D) $-OCF_2-CHCl_2$ I) $-OCH_2-CF_3$
E) $-OCF_2-CHFCl$ M) $-OCF_2-CHF-CF_3$;

$R_a$ für Fluor, Chlor, Brom, Methyl oder für eine beliebige $C_1$-$C_3$ Haloalkoxygruppe vorstehender Bedeutung A) bis I) oder M) steht.

U und V unabhängig voneinander $C_1$-$C_6$ Alkyl darstellen oder zusammen eine der für Formel I genannten Alkylenbrücken bilden, worin

$R_1$ Wasserstoff oder $C_1$-$C_2$ Alkyl und

$R_2$ $C_1$-$C_4$ Alkyl, ein- oder mehrfach durch Fluor oder Chlor substituiertes $C_1$-$C_2$ Alkyl, Phenyl, ein- oder zweifach durch Chlor und/oder Methyl substituiertes Phenyl oder die Gruppe $-CH_2-O-R_6$ bedeuten, bei der $R_6$ $C_1$-$C_4$ Alkyl, ein- bis dreifach durch Fluor oder durch Methoxy substituiertes $C_1$-$C_3$ Alkyl bedeutet oder aber $C_3$-$C_4$ Alkenyl oder Propargyl oder aber Phenyl oder Benzyl ist, deren aromatischer Ring unsubstituiert oder durch Fluor, Chlor, Methyl, Methoxy, Nitro und/oder $CF_3$ ein- oder zweifach substituiert ist, und worin $R_1$ und $R_2$ zusammen auch eine unsubstituierte oder methylsubstituierte Tetramethylenbrücke bilden können; und worin

$R_3$ Wasserstoff

$R_4$ Wasserstoff, Methyl oder Aethyl, und

$R_5$ Wasserstoff, Methyl, Aethyl oder n-Propyl bedeuten, und $R_3$, $R_4$ und $R_5$ zusammen 0 bis 4 Kohlenstoffatome besitzen (Verbindungsgruppe Ic).

Innerhalb der letztgenannten Verbindungsgruppe Ic sind solche Verbindungen der Formel I von Bedeutung, bei denen die $C_1$-$C_3$-Haloalkoxygruppe eine der folgenden Bedeutungen hat:

A) $-OCHF_2$ E) $-OCF_2CHFCl$

B) $-OCF_2-CHF_2$ G) $-OCF_2CFCl_2$

$R_a$ für Fluor, Chlor, Brom, Methyl oder eine beliebige $C_1$-$C_2$ Haloalkoxygruppe vorstehender Bedeutung A), B), E) oder G) steht;

U und V unabhängig voneinander $C_1$-$C_6$-Alkyl darstellen oder zusammen eine der für Formel I genannten Alkylenbrücken bilden, worin

$R_1$ Wasserstoff oder $C_1$-$C_2$-Alkyl und

$R_2$ Wasserstoff, $C_1$-$C_4$-Alkyl, ein- oder mehrfach durch Fluor oder Chlor substituiertes $C_1$-$C_2$-Alkyl, Phenyl, einfach durch Chlor und/oder Methyl substituiertes Phenyl oder die Gruppe $-CH_2OR_6$ bedeuten, bei der $R_6$ $C_1$-$C_4$-Alkyl, ein- bis dreifach durch Fluor oder durch Methoxy substituiertes $C_1$-$C_3$-Alkyl bedeutet oder aber $C_3$-$C_4$-Alkenyl oder Propargyl oder aber Phenyl ist, das unsubstituiert oder durch Fluor, Chlor, Methyl und/oder $CF_3$ monosubstituiert ist, und worin

$R_3$ Wasserstoff,

$R_4$ Wasserstoff, Methyl oder Aethyl und

$R_5$ Wasserstoff, Methyl oder Aethyl bedeuten, und

$R_3$, $R_4$ und $R_5$ zusammen 0 bis 4 Kohlenstoffatome besitzen (Verbindungsgruppe Id).

Innerhalb der letztgenannten Verbindungsgruppe Id sind solche Verbindungen der Formel I von Bedeutung, bei denen die Halogenalkoxygruppe

A) $-OCHF_2$ E) $-OCF_2CHFCl$

B) $-OCF_2CHF_2$ G) $-OCF_2CFCl_2$

bedeutet;

$R_a$ für Fluor, Chlor, Brom, Methyl, $OCHF_2$ oder $-OCF_2CHF_2$ steht;

U und V unabhängig voneinander $C_1$-$C_6$-Alkyl sind oder eine der in Formel I dargestellten Alkylenbrücken bilden, worin

$R_1$ Wasserstoff oder Methyl und

$R_2$ Wasserstoff, $C_1$-$C_3$-Alkyl, ein- oder mehrfach durch Fluor oder Chlor substituiertes $C_1$-$C_2$-Alkyl, Phenyl, einfach durch Chlor substituiertes Phenyl oder die Gruppe $-CH_2OR_6$ bedeuten, wobei $R_6$ $C_1$-$C_3$-Alkyl, ein bis dreifach durch Fluor substituiertes $C_1$-$C_2$-Alkyl oder $C_3$-$C_4$-Alkenyl oder Propargyl oder Phenyl darstellt, und worin

$R_3$ Wasserstoff, $R_4$ Wasserstoff, Methyl oder Aethyl und $R_5$ Wasserstoff oder Methyl bedeuten und $R_3$, $R_4$ und $R_5$ zusammen 0 bis 2 Kohlenstoffatome besitzen (Verbindungsgruppe Ie).

Die Verbindungen der Formel I werden hergestellt, indem man

A) ein Triazol der Formel II

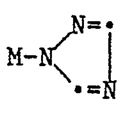

(II),

worin M Wasserstoff oder ein Metallkation darstellt, mit einer Verbindung der Formel III

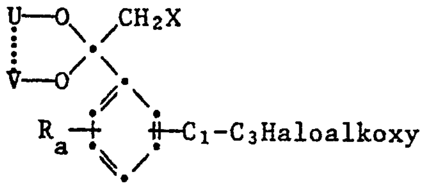

(III),

worin X eine nukleophile Abgangsgruppe bedeutet, kondensiert oder indem man
B) in einer Verbindung der Formel IV

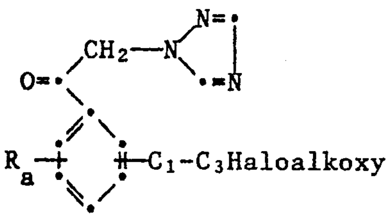

(IV)

die Carbonylfunktion in eine Ketalfunktion der Formel

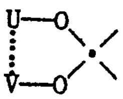

(V)

überführt, oder indem man
C) zur Herstellung von Verbindungen der Formel I, worin U und V gemeinsam eine Gruppe der Formel $-CH_2CH(CH_2ZR_6')-$ darstellen und $R_6'$ einen von Wasserstoff verschiedenen Rest $R_6$ bedeutet, Verbindungen der Formeln VI und VII miteinander kondensiert,

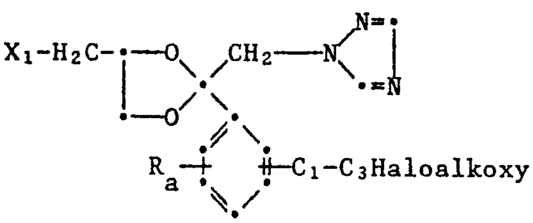

und $R_6-X_2$ (VII)

(VI)

worin einer der Reste $X_1$ und $X_2$ gegebenenfalls in Salzform vorliegendes Hydroxy oder Mercapto, z.B. der Formel -Z-M, und der andere eine beliebige nukleophile Abgangsgruppe X bedeutet oder sowohl $X_1$ als auch $X_2$ Hydroxygruppen darstellen, oder indem man
D) Hydrazine der Formel IX,

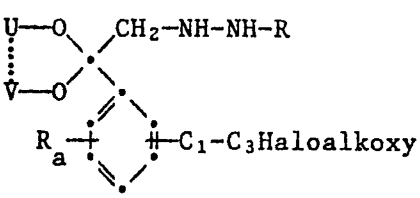

(IX),

a) mit R = -CHO, -COR', -COOR' oder $-CONH_2$ und R' = $C_1$-$C_4$-Alkyl, Benzyl oder Phenyl, hydrolysiert und die entstandene Verbindung der Formel IX mit R = H oder ihre Salze mit anorganischen oder organischen Säuren mit Hilfe von Formamid und/oder [3-(Dimethylamino)-2-azaprop-2-en-1-yliden]-dimethylammonium-chlorid (Azasalz), $[(CH_3)_2N \overset{+}{=} CH\text{-}N{=}CH\text{-}N(CH_3)_2]Cl^-)$, in die Verbindung der Formel I überführt, oder b)

Verbindungen der Formel IX mit R = -COR′ mit wässriger Ameisensäure in die N,N′-Bisformylderivate überführt und diese mit Formamid, gegebenenfalls in Gegenwart von $NH_3$ oder einer $NH_3$ liefernden Substanz zu einem Triazol-Derivat der Formel I cyclisiert, wobei in Formel IX R Wasserstoff, -CHO, -COR′, -COOR′ oder $-CONH_2$ und R′ $C_1$-$C_4$- Alkyl, Benzyl oder Phenyl bedeuten, und, falls erwünscht, eine verfahrensgemäss erhaltene Verbindung in eine andere Verbindung der Formel I umwandelt und/oder eine verfahrensgemäss erhaltene freie Verbindung in ein Säureadditionssalz, oder ein verfahrensgemäss erhaltenes Säureadditionssalz in die freie Verbindung oder in ein anderes Säureadditionssalz, oder eine verfahrensgemäss erhaltene freie Verbindung bzw. ein verfahrensgemäss erhaltenes Säureadditionssalz in einen Metallkomplex überführt; wobei die Substituenten in den obigen Formeln die unter Formel I angegebenen Bedeutungen haben.

Metallkationen M sind dabei beispielsweise Alkalimetall-, z.B. Lithium-, Natrium- oder Kaliumkationen, oder Erdalkalimetall-, z.B. Magnesium-, Calcium-, Strontium- oder Bariumkationen.

Die weiter oben angegebenen nukleophilen Abgangsgruppen sind beispielsweise reaktionsfähige veresterte Hydroxygruppen, wie die mit einer Halogenwasserstoffsäure, (z.B. Fluor-, Chlor-, Brom- oder Jodwasserstoffsäure), oder wie die mit einer Niederalkan-, gegebenenfalls substituierten Benzol- oder Halogensulfonsäure, z.B. sind mit Methan-, Ethan-, Benzol-, p-Toluol- oder Fluorsulfonsäure veresterte Hydroxygruppen zu nennen.

Variante A: Die Umsetzung eines Triazols der Formel II,

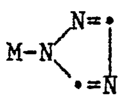

(II),

worin M vorzugsweise ein Metallatom, insbesondere ein Alkalimetallatom darstellt, wird mit einer Verbindung der Formel III

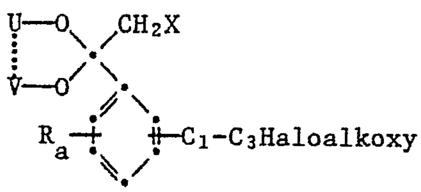

(III),

worin $R_a$, U und V die unter Formel I angegebenen Bedeutungen haben und X eine Abgangsgruppe, z.B. Halogen, insbesondere Chlor, Brom oder Jod, oder Benzolsulfonyloxy, p-Tosyloxy, Trifluoracetyloxy oder bevorzugt Niederalkylsulfonyloxy wie z.B. Mesyloxy, bedeutet, bevorzugt in einem relativ polaren, jedoch reaktionsinerten organi schen Lösungsmittel durchgeführt, z.B. N,N-Dimethylformamid, Hexamethylphosphorsäuretriamid (HMPT) N,N-Dimethylacetamid, Dimethylsulfoxid, Acetonitril, Benzonitril und anderen. Derartige Lösungsmittel können in Kombination mit anderen reaktionsinerten Lösungsmitteln, wie aliphatischen oder aromatischen Kohlenwasserstoffen, z.B. Benzol, Toluol, Xylol, Hexan, Petrolether, Chlorbenzol, Nitrobenzol u.a. verwendet werden.

Bedeutet X Chlor oder Brom, so kann man zweckmässig Alkalijodid (wie NaJ oder KJ) zur Beschleunigung der Reaktion zusetzen. Erhöhte Temperaturen von 0 bis 220°C, bevorzugt 80-170°C, sind vorteilhaft. Zweckmässig wird das Reaktionsgemisch unter Rückfluss erhitzt.

In den Fällen, in denen in Formel II M für Wasserstoff steht, wird das Verfahren in Gegenwart einer Base durchgeführt. Beispiele solcher Basen sind anorganische Basen wie ie Oxide, Hydroxide, Hydride, Carbonate und Hydrogencarbonate von Alkali und Erdalkalimetallen sowie z.B. tert. Amine wie Triethylamin, Triethylendiamin, Piperidin, Pyridin, 4-Dimethylaminopyridin, 4-Pyrrolidylpyridin usw.

Bei dieser und den folgenden Herstellungsvarianten können die Zwischen- und Endprodukte aus dem Reaktionsmedium isoliert und, falls erwünscht, auf eine der allgemein üblichen Methoden gereinigt werden, z.B. durch Extraktion, Kristallisation, Chromatographie, Destillation usw.

Variante B: Die Ueberführung der Carbonylgruppe in Verbindungen der Formel IV in die Gruppe der Formel V erfolgt durch Umsetzung mit einem Orthocarbonsäure-tri-$C_1$-$C_{12}$-alkylester, dessen $C_1$-$C_{12}$-Alkylgruppen gegebenenfalls durch Halogen oder $C_1$-$C_6$-Alkoxy substituiert sind, oder in Gegenwart einer Säure mit mindestens 2 Mol eines einwertigen Alkohols der Formel U-OH (Va), wobie Verbindungen der Formel I erhalten werden, worin U und V gleiche, gegebenenfalls substituierte $C_1$-$C_{12}$-Alkylgruppen bedeuten, oder durch Umsetzung mit einem Diol der Formel Vb

HO-U—V-OH (Vb),

wobei Verbindungen der Formel I erhalten werden, worin U und V gemeinsam eine der eingangs definierten Alkylenbrücken darstellt. Dabei haben Y, $R_a$, U und V die unter Formel I angegebenen Bedeutungen.

Diese Ketalisierungsreaktion kann analog zu bereits bekannten Ketalisierungs-Reaktionen durchgeführt werden, beispielsweise analog zur Herstellung von 2-Brommethyl-2,4-diphenyl-1,3-dioxolan [Synthesis, 1974 (I), 23].

Bei der bevorzugten Ausführung der Ketalisierung werden beide Reaktionspartner mehrere Stunden zusammen mit einem Azeotrop-Bildner in einem der üblichen organischen Lösungsmittel unter Rückfluss erhitzt. Als Azeotrop-Bildner kommen z.B. Benzol, Toluol, Xylol, Chloroform oder Tetrachlorkohlenstoff in Frage, wobei zur Beschleunigung der Reaktion ein Zusatz einer starken Säure, wie z.B. p-Toluolsulfonsäure, vorteilhaft sein kann. Verwendbare organische Lösungsmittel sind in diesem Fall z.B. aromatische Kohlenwasserstoffe, wie Benzol, Toluol, Xylol usw., gesättigte Kohlenwasserstoffe wie z.B. 1,1,1-Trichlorethan.

Es sind auch weitere Wege der Ketalisierung durchführbar, z.B. durch Umketalisierung. Man kann ein Keton IV, das mit einem von den Alkanolen bzw. Diolen der Formeln Va bzw. Vb verschiedenen Alkohol oder Phenol bereits ketalisiert ist, durch Reaktion mit überschüssigem Alkanol Va bzw. dem Diol Vb zu (I) umketalisieren. Das Ausgangsprodukt ist z.B. gemäss der Verfahrensvariante A) zugänglich.

Variante C: Die Herstellung von Verbindungen der Formel I, worin Substituenten U und V gemäss Variante C) zusammen für $-CH_2-CH(CH_2ZR_6)-$ stehen, erfolgt beispielsweise durch Reaktion einer Verbindung der Formel VI mit einer Verbindung der Formel VII, worin $X_1$ eine Gruppe -OH oder -SH und $X_2$ eine nucleophile Abgangs gruppe X bedeutet. Die Reaktion wird bevorzugt in reaktions-inerten organischen Lösungsmitteln durchgeführt. Es eignen sich hierzu z.B. N,N-Dimethylformamid, N,N-Dimethylacetamid, Hexamethylphosphortriamid, Dimethylsulfoxid, 4-Methyl-3-pentanon usw. Auch Gemische mit anderen reaktionsinerten Lösungsmitteln, z.B. mit aromatischen Kohlenwasserstoffen wie Benzol, Toluol, Xylol usw. können verwendet werden. In manchen Fällen kann es sich als vorteilhaft erweisen, zur Beschleunigung der Reaktionsgeschwindigkeit in Gegenwart einer Base zu arbeiten. Solche Basen sind z.B. Alkalimetallhydride oder Alkalimetallcarbonate. In gewissen Fällen kann es auch von Vorteil sein, dass man die Verbindung VI zuerst auf bekannte Art in ein geeignetes Metall-Salz überführt.

Dies geschieht vorzugsweise durch Reaktion von VI mit einer Na-Verbindung, z.B. Natriumhydrid, Natriumhydroxid usw.. Anschliessend wird dieses Salz von VI mit der Verbindung der Formel VII umgesetzt. Zur Erhöhung der Reaktionsgeschwindigkeit kann in manchen Fällen auch bei erhöhter Temperatur, vorzugsweise 80°C bis 130°C bzw. am Siedepunkt des Lösungsmittels, gearbeitet werden.

In analoger Weise kann man auch Verbindungen der Formeln VI und VII, worin $X_1$ eine Abgangsgruppe X und $X_2$ eine Gruppe -OH oder -SH ist, umsetzen.

Bei der zu Produkten der Formel I, worin Z Sauerstoff ist, führenden Kondensations-Reaktion von Verbindungen der Formeln VI und VII, worin $X_1$ und $X_2$ Hydroxy darstellen, können die Reaktanden in einem geeigneten Lösungsmittel unter Rückfluss erhitzt werden, wobei gleichzeitig das entstehende Wasser azeotrop aus dem Reaktionsgemisch abdestilliert wird. Als Lösungsmittel kommen aromatische Kohlenwasserstoffe, wie Toluol oder der Alkohol $HO-R_6$ selbst in Frage. Bei dieser Reaktion arbeitet man zweckmässigerweise in Gegenwart einer starken Säure, z.B. p-Toluolsulfonsäure.

Variante D: Hydrazine der Formel IX, worin R ungleich Wasserstoff ist, werden vor dem Ringschluss in an sich bekannter Weise in Gegenwart von Säuren oder Basen zu Verbindungen der Formel IX mit R = H oder Salzen davon hydrolysiert. Als Basen können z.B. Alkalimetall- oder Erdalkalimetallhydroxide oder -carbonate verwendet werden, wie Natrium-, Kalium- und Calciumhydroxid oder entsprechende Carbonate. Vorzugsweise wird die Hydrolyse in Gegenwart einer starken Säure, besonders einer anorganischen Säure, wie HCl, Schwefelsäure oder Phosphorsäure, durchgeführt. Die Hydrolyse kann in wässrigem oder wässrig-organischem Medium, wie Wasser/Alkanol-Gemischen, besonders Gemischen aus Wasser und Methanol oder Ethanol, erfolgen.

Für den Ringschluss gemäss Verfahrensvariante a) werden das Formamid und/oder das [3-(Dimethylamino)-2-azaprop-2-en-1-yliden]-dimethylammoniumchlorid zweckmässig in mindestens äquimolekularer Menge, bezogen auf die Verbindung der Formel IX (R = H) verwendet. Dabei wird die Umsetzung mit Vorteil in Gegenwart eines inerten organischen Lösungsmittels vorgenommen, z.B. Alkanolen, Estern, Ethern oder Amiden der vorerwähnten Art, Alkylnitrilen mit 2-5 C-Atomen, wie Acetonitril, Propionitril, Butyronitril; ferner Benzonitril; 3-Alkoxypropionitrilen mit 1 oder 2 C-Atomen im Alkoxyteil, wie 3-Methoxypropionitril und 3-Ethoxypropionitril. Bevorzugte Lösungsmittel für die Umsetzung mit dem Azasalz sind $C_1$-$C_5$-Alkanole, besonders Ethanol. Bei der Umsetzung mit Formamid wird vorzugsweise überschüssiges Formamid als Lösungsmittel eingesetzt. Die Reaktionstemperatur für den Ringschluss gemäss a) liegen im allgemeinen zwischen 20° und 200°C, bevorzugt zwischen 20° und 180°C.

Für die Formylierung von Verbindungen der Formel IX mit R = COR', z.B. $-COCH_3$, $-COC_2H_5$ oder $-COC_3H_7$, gemäss Verfahrensvariante b) wird zweckmässig 85%ige wässrige Ameisensäure eingesetzt. Die Reaktionstemperaturen liegen dabei bevorzugt zwischen 70° und 100°C. Für den Ringschluss der N,N'-Bisformylderivate wird das Formamid zweckmässig in mindestens äquimolekularer Menge, bezogen auf die Verbindung der Formel IX (R = -COR') verwendet. Als $NH_3$ liefernde Substanzen kommen vor allem Salze von Ammoniak mit schwachen Säuren, z.B. Carbonsäuren, in Betracht. Bevorzugte Salze sind Ammoniumcarbonat, -bicarbonat oder -formiat. Die Reaktionstemperaturen für den Ringschluss der N,N'-Bisformylderivate liegen im allgemeinen zwischen 50° und 200°C, bevorzugt zwischen 120° und 180°C.

Bei allen beschriebenen Ketalisierungs-Reaktionen eines Ketons mit einem substituierten $\alpha,\beta$- oder $\alpha,\gamma$-Diol entstehen vorwiegend Gemische von Diastereomeren der Endprodukte I. Die Verbindungen der Formel I können beispielsweise in nachfolgenden beiden diastereomeren Formen, nämlich A- und B-Typen, vorliegen:

(X) 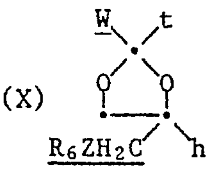 A-Typen 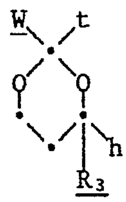 (XI)

Die Konfiguration vom Typ A soll hier und im folgenden als das "trans"-Isomere oder "trans-Racemat", bezeichnet werden,

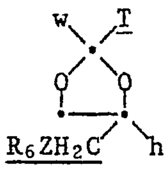 B-Typen 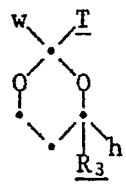

wobei w bzw. W für die Gruppe

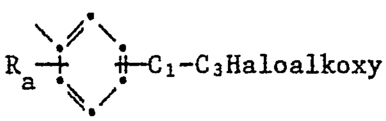

und t bzw T für die Gruppe

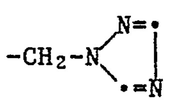

steht und die unterstrichenen Symbole W, T, $R_3$ und $R_6ZH_2C$- in der räumlichen Darstellung für Gruppierungen vor der Zeichenebene und die kleingeschriebenen Symbole, t, w und h (= Wasserstoff) für Gruppierungen hinter der Zeichenebene stehen. Die Konfiguration vom Typ B soll entsprechend als das "cis"-Isomere, "cis-Racemat", bezeichnet werden. Die Trennung der beiden Diastereomeren kann beispielsweise durch fraktionierte Kristallisation oder durch Chromatograpie (Dünnschicht-, Dickschicht-, Säulenchromatographie, Flüssigkeitschochdruckchromatographie usw.) erfolgen. Die Herstellung der optisch reinen Isomeren ist ebenfalls möglich. Als Beispiel diene die weiter unten genannte Verbindung 1.3.

Ausgehend von den beiden optisch aktiven 1,2-Butandiolen lassen sich die vier Isomeren folgendermassen herstellen:

a)

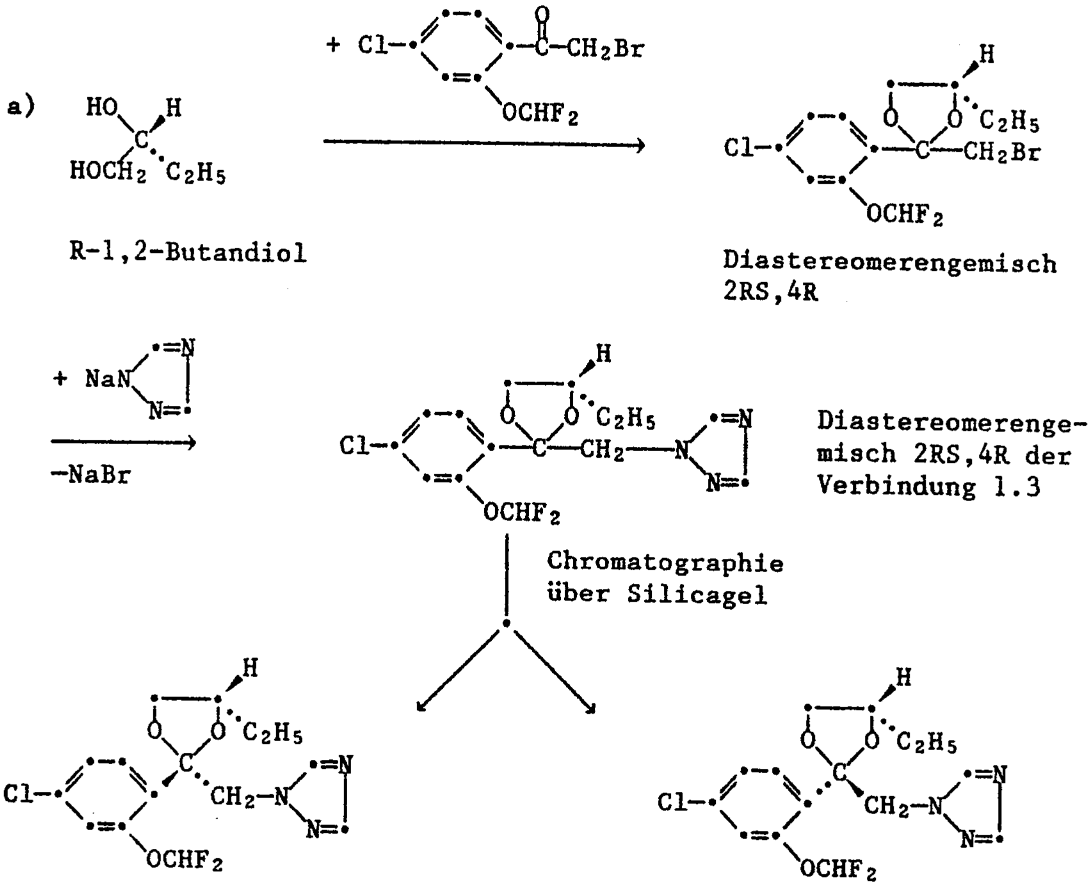


R-1,2-Butandiol

Diastereomerengemisch 2RS,4R

Diastereomerengemisch 2RS,4R der Verbindung 1.3

2S,4R-Isomeres

2R,4R-Isomeres

b)

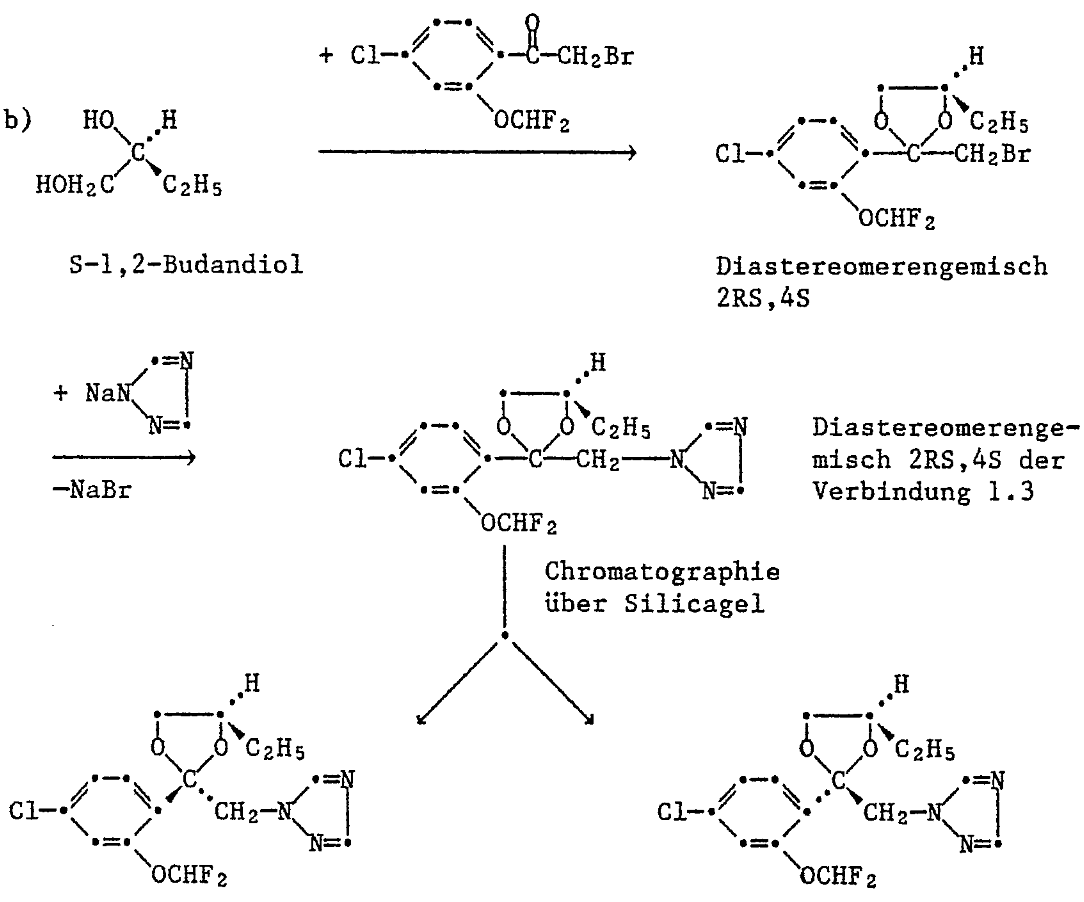


S-1,2-Budandiol

Diastereomerengemisch 2RS,4S

Diastereomerengemisch 2RS,4S der Verbindung 1.3

2S,4S-Isomeres

2R,4S-Isomeres

Die vier Isomeren unterscheiden sich in ihren fungiziden Aktivitäten, wobei diese Unterschiede von den verschiedenen Pilzgattungen abhängig sind. Hierbei ist die 2S-Konfiguration mit der cis-Anordnung von Ethyl und Triazolylmethyl (2S,4R-Isomeres) dies etwas aktivere Form.

Auch die beiden "trans- und cis"-Racemate zeigen unterschiedliche biologische Wirkungen. Im allgemeinen werden für praktische Zwecke die bei der Herstellung anfallenden Diastereomerengemische als solche verwendet.

Die Erfindung betrifft sämtliche isomeren Verbindungen der Formel I in reiner Form oder in beliebigem Zahlenverhältnis zueinander, sowie ihre Salze und Metallsalzkomplexe.

Das Herstellungsverfahren von Verbindungen der Formel I ist in seinen beschriebenen Verianten A, B, C und D ein Bestandteil dieser Erfindung.

Die Zwischenprodukte der Formel III, worin $R_a$, U und V die unter Formel I angegebenen Bedeutungen haben und X Halogen, Benzolsulfonyloxy, p-Tosyloxy, Trifluoroacetyloxy oder Niederalkylsulfonyloxy bedeutet, und Verfahren zu ihrer Herstellung sind ein weiterer Gegenstand vorliegender Erfindung.

Verbindungen der Formel III lassen sich durch Ketalisierung eines entsprechend substituierten Acetophenons der Formel XII

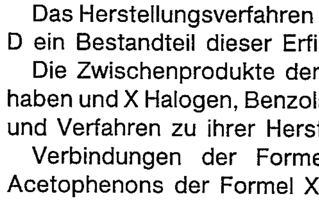

(XII)

gewinnen. Die Ketalisierung erfolgt mit einem einwertigen Alkohol U-OH (bzw. V-OH) oder mit einem zweiwertigen Alkohol HO-U-V-OH, wobei $R_a$, X, U und V die für Formel I gegebene Bedeutung haben, in Gegenwart einer starken Säure wie Toluolsulonsäure in inerten Lösungsmitteln wie Kohlenwasserstoffen.

Die Zwischenprodukte der Formel XII lassen sich nach bekannten Methoden herstellen, indem z.B. in der Seitenkette des (aus Haloalkan, wie z.B Freon, und 2-Hydroxy- und/oder 4-Hydroxyacetophenon-Derivat gewonnenen) $C_1$-$C_3$-Haloalkoxyacetophenon-Derivates mit z.B. Brom halogeniert wird.

Die als Zwischenprodukte der Formel IV dienenden Ketone sind ein weiterer Gegenstand vorliegender Erfindung.

Es wurde überraschend festgestellt, dass Verbindungen der Formel I ein für praktische Bedürfnisse sehr günstiges Mikrobizid-Spektrum gegen phytopathogene Pilze und Bakterien aufweisen. Sie besitzen sehr vorteilhafte kurative, präventive und systemische Eigenschaften und lassen sich zum Schutz von Kulturpflanzen einsetzen. Mit den Wirkstoffen der Formel I können an Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder vernichtet werden, wobei auch später zuwachsende Pflanzenteile von derartigen Mikroorganismen verschont bleiben.

Die Wirkstoffe sind gegen die den folgenden Klassen angehörenden phytopathogenen Pilze wirksam: Ascomyceten (z.B. Venturia, Podosphaera, Erysiphe, Monilinia, Uncinula); Basidiomyceten (z.B. die Gattungen Hemileia, Rhizoctonia, Puccinia); Fungi imperfecti (z.B. Botrytis, Helminthosporium, Fusarium, Septoria, Cercospora, Alternaria und insbesondere Pyricularia). Ueberdies wirken die Verbindungen der Formel I systemisch. Sie können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden. Die erfindungsgemässen Wirkstoffe zeichnen sich durch besonders gute Pflanzenverträglichkeit und durch ihre Umweltfreundlichkeit aus, da sie bereits nach einer Anbausaison nicht mehr im Boden nachweisbar sind. Auffallend ist darüber hinaus die ausgeprägte Aktivität der vorliegenden Wirkstoffgruppe gegen Reiskrankheiten wie z.B. Pyricularia und Pellicularia. Der Vorteil der Wirkstoffe der Formel I besteht vor allem darin, dass sie einen bereits eingetretenen Krankheitsbefall unerwartet nachdrücklich bekämpfen können (kurative Wirkung). Diese Eigenschaft hat in der Praxis den ausserordentlichen Vorteil, dass Pflanzenkulturen wie Getreide und Reis nicht prophylaktisch mit Wirkstoffen behandelt werden müssen, sondern dass mit der Behandlung solange gewartet werden kann, bis erste Krankheitszeichen von z.B. Pyricularia an Reispflanzen auftreten.

Die Erfindung betrifft somit auch mikrobizide Mittel sowie die Verwendung der Verbindungen der Formel I zur Bekämpfung phytopathogener Mikroorganismen, insbesondere pflanzenschädigender Pilze, bzw. die präventive Verhütung eines Befalls an Pflanzen.

Darüber hinaus schliesst die vorliegende Erfindung auch die Herstellung agrochemischer Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der Verbindungen der Formel I bzw. der neuen Mittel auszeichnet.

Als Zielkulturen für die hierin offenbarten Indikationsgebiete gelten im Rahmen dieser Erfindung z.B. folgende Pflanzenarten: Getreide: (Weizen, Gerste, Roggen, Hafer, Reis, Sorghum und Verwandte); Rüben: (Zucker- und Futterrüben); Kern-, Stein- und Beerenobst: (Aepfel, Birnen, Pflaumen, Pfirsiche, Mandeln, Kirschen, Erd-, Him- und Brombeeren); Hülsenfrüchte: (Bohnen, Linsen, Erbsen, Soja); Oelkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusfrüchte: (Orangen, Zitronen, Grapefruit, Mandarinen); Gemüsesorten: (Spinat, Kopfsalat, Spargel, Kohlarten, Möhren, Zwiebeln, Tomaten, Kartoffeln, Paprika); Lorbeergewächse: (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Blumen, Sträucher, Laubbäume und Nadelbäume wie Koniferen). Diese Aufzählung stellt keine Limitierung dar.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffes der Formel I bzw. eines agrochemischen Mittels, das mindestens einen dieser Wirkstoffe enthält, ist das Aufbringen auf die oberirdischen Pflanzenteile, vor allem das Blattwerk (Blattapplikation). Anzahl der Applikationen und Aufwandmenge richten sich nach den biologischen und klimatischen Lebensbedingungen für den Erreger (Pilzsorte). Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt z.B. in Form von Granulat (Bodenapplikation). Die Verbindungen der Formel I können auch auf Samenkörner aufgebracht werden (Coating), indem man die Körner entweder in einer flüssigen Zubereitung des Wirkstoffs tränkt oder sie mit einer festen Zubereitung beschichtet. Darüberhinaus sind in besonderen Fällen weitere Applikationsarten möglich, z.B. die gezielte Behandlung der Knospen oder der Fruchtstände.

Die Verbindungen der Formel I werden dabei in unveränderter Form oder vorzugsweise zusammen mit den in der Formulierungstechnik üblichen Hilfsmittel eingesetzt und werden daher z.B. zu Emulsionskonzentraten, streichfähigen Pasten, direkt versprühbaren oder verdünnbaren Lösungen, verdünnten Emulsionen,

Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha; bevorzugt 100 g bis 2 kg AS/ha, insbesondere bei 100 g bis 600 g AS/ha.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden. Besonders vorteilhafte, applikationsfördernde Zuschlagstoffe, die zu einer starken Reduktion der Aufwandmenge führen können, sind ferner natürliche (tierische oder pflanzliche) oder synthetische Phospholipide aus der Reihe der Kephaline und Lecithine, wie z.B. Phosphatidylethanolamin, Phosphatidylserin, Phosphatidylglycerin, Lysolecithin, Plasmalogene oder Cardiolipin, die man beispielsweise aus tierischen oder pflanzlichen Zellen, insbesondere aus Hirn, Herz, Leber, Eidotter oder Sojabohnen gewinnen kann. Verwendbare Handelsmischungen sind z.B. Phosphatidylchlorin-Mischungen. Synthetische Phospholipide sind z.B. Dioctanoylphosphatidylcholin und Dipalmitoylphophatidylcholin.

Als oberflächenaktive Verbindungen kommen je nach Art des zu formulierenden Wirkstoffes der Formel I nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyllaurinsalze zu erwähnen.

Häufiger werden jedoch sog. synthetische Tenside verwendet, insbesondere Fettsulfonate, Fettsulfate, sulfonierte Benzimidazolderivate oder Alkylsulfonate.

Die Fettsulfonate oder -sulfate liegen in der Regel als Alkali-, Erdalkali- oder gegebenenfalls substituierte Ammoniumsalze vor und weisen einen Alkalirest mit 8 bis 22 C-Atomen auf, wobei Alkyl auch den Alkylteil von Acylresten einschliesst, z.B. Das Na- oder Ca-Salz der Ligninsulfonsäure, des Dodecylschwefelsäureesters oder eines aus natürlichen Fettsäuren hergestellten Fettalkoholsulfatgemisches.

Hierher gehören auch die Salze der Schwefelsäureester und Sulfonsäuren von Fettalkohol-Ethylenoxyd-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die in der Formulierungstechnik gebräuchlichen Tenside sind dem Fachmann aus der Fachliteratur bekannt:

Die agrochemischen Zubereitungen enthalten in der Regel 0,1 bis 99%, insbesondere 0,1 bis 95% Wirkstoff der Formel I, 99,9 bis 1%, insbesondere 99,8 bis 5% eines festen oder flüssigen Zusatzstoffes und 0 bis 25%, insbesondere 0,1 bis 25% eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Die nachfolgenden Beispiele dienen zur näheren Erläuterung der Erfindung, ohne dieselbe einzuschränken. Prozente und Teile beziehen sich auf das Gewicht. Temperaturen sind in Celsiusgraden angegeben. Darüber hinaus bedeutet RT = Raumtemperatur; $C_6H_5$ ist eine Phenylgruppe.

1. Herstellungsbeispiele

Beispiel 1.1: Herstellung von

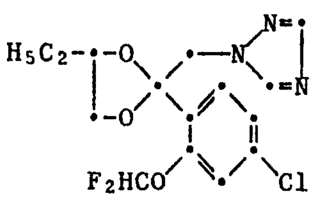

(Verb. 1.3)

2-[2'-Difluormethoxy-4'-chlor-phenyl]-2-(1H-1,2,4-triazolylmethyl)-4-ethyl-1,3-dioxolan

A) Synthese der Zwischenprodukte

a) Herstellung von

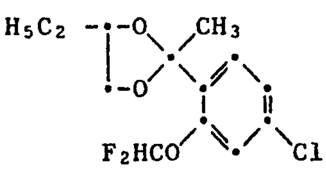

2-[2'-Difluormethoxy-4'-chlor-phenyl]-2-methyl-4-ethyl-1,3-dioxolan

12,8 Teile 1-(4-Chlor-2-difluormethoxy-phenyl)-ethanon-(1) und 10,4 Teile 1,2-Butandiol in 100 ml abs. Toluol werden in Gegenwart von 0,2 Teilen katalytisch wirkenden p-Toluolsulfonsäure 12 Std. am Wasserabscheider unter Rückfluss erhitzt. Nach dem Abkühlen auf RT wird das Reaktionsgemisch zweimal mit je 100 ml Wasser gewaschen, über Natriumsulfat getrocknet, filtriert und das Lösungsmittel verdampf. Das zurückbleibende Oel wird durch Destillation im Hochvakuum gereinigt; Sdp. 78-80°C/0,04 mbar.

b) Herstellung von

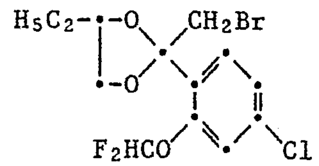

2-[2'-Difluormethoxy-4'-chlor-phenyl]-2-brommethyl-4-ethyl-1,3-dioxolan

12,7 Teile des unter a) hergestellten 2-(4'-Chlor-2'-difluormethoxyphenyl)-2-methyl-4-ethyl-1,3-dioxolans werden in 100 ml abs. Chloroform gelöst und auf 30°C erwärmt. Unter Belichtung mittels einer 150 Watt Spotlampe werden 7,3 Teile Brom in 30 ml abs. Chloroform innerhalb 1 Std. zugetropft und anschliessend noch 2 Std. bei RT weitergerührt. Das Reaktionsgemisch wird zweimal mit je 100 ml Wasser gewaschen, über Natriumsulfat getrocknet, filtriert und das Lösungsmittel verdampft. Der ölige Rückstand wird durch Hochvakuumdestillation gereinigt;
Sdp. 113-115°C/0,05 mbar.

B) Synthese des Endproduktes

5,9 Teile 1,2,4-Triazol-Natriumsalz und eine katalytisch wirkende Menge Kaliumjodid werden in 100 ml Dimethylformamid mit 12 Teilen des nach b) hergestellten 2-(2'-Difluormethoxy-4'-chlor-phenyl)-2-brommethyl-4-ethyl-1,3-dioxolans 16 Std. bei einer Badtemperatur von 130° gerührt. Nach dem Abkühlen auf Raumtemperatur wird das Reaktionsgemisch auf 500 ml Wasser gegossen, dreimal mit je 200 ml Diethylether extrahiert, die vereinigten organischen Phasen zweimal mit je 200 ml Wasser gewaschen, über Natriumsulfat getrocknet, filtriert und das Lösungsmittel verdampft. Der ölige Rückstand wird über eine 50 cm lange Kieselgelsäule mittels Methylenchlorid/Diethylether (1:1) chromatographiert. Nach Verdampfen des Laufmittels wird ein gelbes Oel erhalten, $n_D^{27}$ : 1,5132.

<u>Beispiel 1.2</u>: Herstellung von

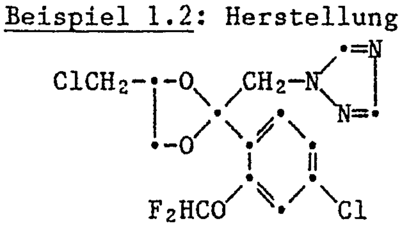

(Verb. 1.59)

2-[2'-Difluoromethoxy-4'-chlor-phenyl]-2-[1H-1,2,4-triazolylmethyl]-4-chlormethyl-1,3-dioxolan

14,2 Teile 1-(4-Chlor-2-difluormethoxy-phenyl)-2-(1H-1,2,4-triazolyl)-ethanon-(1) und 100 ml n-Butanol in 450 ml abs. Toluol werden in Gegenwart von 9,4 Teilen p-Toluolsulfonsäure 5 Std. am Wasserabscheider unter Rückfluss erhitzt. Nach dem Abkühlen auf 60° werden 33 Teile 3-Chlor-1,2-propandiol und nochmals 18 Teile p-Toluolsulfonsäure zugegeben und 80 Std. am Wasserabscheider unter Rückfluss erhitzt. Nach dem Abkühlen auf RT wird das Reaktionsgemisich zweimal mit gesättigter, wässeriger Kaliumcarbonatlösung und zweimal mit je 200 ml Wasser gewaschen, über Natriumsulfat getrocknet, filtriert und das Lösungsmittel am Rotationsverdampfer verdampft. Der ölige Rückstand wird über eine 50 cm lange Kieselgelsäule mit Methylenchlorid/Diethylether (1:1) als Laufmittel chromatographiert. Nach dem Verdampfen des Laufmittelgemisches wird das Titelprodukt als hellgelbes Oel erhalten; $n_D^{22}$ :1.5336.

Es lassen sich die nachfolgenden Zwischenprodukte der Formel IV einsetzen:

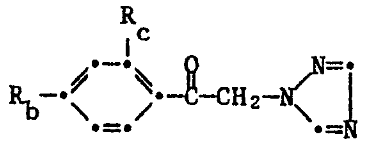

(IV)

| $R_c$ | $R_b$ | Physikalische Konstante |
|---|---|---|
| $-OCHF_2$ | Cl | Smp. 97-102° |
| Cl | $-OCHF_2$ | Smp. 67-69° |
| $CH_3$ | $-OCHF_2$ | Smp. 52-57° |
| $-OCHF_2$ | Br | Smp. 85-92° |
| $-OCHF_2$ | F | Smp. 106-113° (Zers.) |

Auf analoge Weise lassen sich auch die nachfolgenden Zwischenprodukte (falls nicht besonders vermerkt, sind es Diastereomerengemische mit unterschiedlichen Mischungsverhältnissen) der Formel III herstellen:

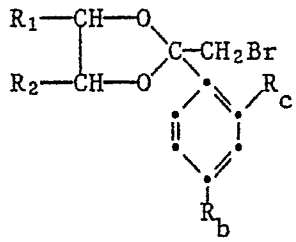

(III)

| $R_1$ | $R_2$ | $R_c$ | $R_b$ | Physikalische Konstante |
|---|---|---|---|---|
| $-C_3H_7$-n | H | $-OCHF_2$ | Br | Sdp: 124-126°/ 0,002 mbar |
| $-C_2H_5$ | H | $CH_3$ | $-OCF_2CHCl_2$ | $n_D^{24}$: 1.5171 |
| $-C_3H_7$-n | H | $CH_3$ | $-OCF_2CCl_2F$ | $n_D^{23}$: 1.4981 |
| $CH_3$ | $CH_3$ | $-OCHF_2$ | Br | Sdp: 114-115°/ 0,02 mbar |
| $-C_3H_7$-n | H | $CH_3$ | $-OCF_2CHCl_2$ | $n_D^{24}$: 1.5145 |
| 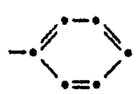 | H | $-OCHF_2$ | Cl | Sdp: 143-149°/ 0,002 mbar |
| $CH_3$ | H | $CH_3$ | $-OCF_2CHClF$ | $n_D^{24}$: 1.5024 |
| $-C_2H_5$ | H | $CH_3$ | $-OCF_2CHF_2$ | $n_D^{24}$: 1.4828 |
| $CH_3$ | H | $-OCHF_2$ | Br | $n_D^{28}$: 1.5212 |
| $-C_3H_7$-n | H | $CH_3$ | $-OCF_2CHF_2$ | $n_D^{25}$: 1.4813 |
| $-C_2H_5$ | H | $CH_3$ | $-OCF_2CHClF$ | $n_D^{24}$: 1.5002 |

| $R_1$ | $R_2$ | $R_c$ | $R_b$ | Physikalische Konstante |
|---|---|---|---|---|
| $-CH_2OCH_3$ | H | $CH_3$ | $-OCF_2CHF_2$ | $n_D^{24}$: 1.4848 |
| $-CH_2OCH_2CF_3$ | H | $-OCHF_2$ | Br | Sdp: 118-121°/ 0,02 mbar |
| $CH_3$ | H | $CH_3$ | $-OCF_2CHFCF_3$ | $n_D^{24}$: 1.4655 |
| $-C_3H_7$-n | H | $CH_3$ | $-OCF_2CHClF$ | $n_D^{24}$: 1.4972 |
| $-C_2H_5$ | H | $CH_3$ | $-OCF_2CHFCF_3$ | $n_D^{24}$: 1.4649 |
| $CH_3$ | H | $CH_3$ | $-OCHF_2$ | $n_D^{23}$: 1.5142 |
| $-C_3H_7$-n | H | $CH_3$ | $-OCHF_2$ | $n_D^{23}$: 1.5056 |
| $-CH_2OC_4H_9$-sek. | H | $-OCHF_2$ | Cl | $n_D^{28}$: 1.5011 |
| $CH_3$ | $CH_3$ | $CH_3$ | $-OCHF_2$ | $n_D^{23}$: 1.5086 |
| $-C_3H_7$-n | H | $CH_3$ | $-OCF_2CHFCF_3$ | $n_D^{24}$: 1.4635 |
| 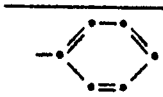 | H | $-OCHF_2$ | Br | $n_D^{26}$: 1.5676 |
| $-CH_2OCH_3$ | H | $CH_3$ | $-OCF_2CHClF$ | $n_D^{25}$: 1.5102 |
| $-CH_2OCH_2CF_3$ | H | $-OCHF_2$ | Cl | $n_D^{28}$: 1.4844 |
| $CH_3$ | H | $CH_3$ | $-OCF_2CCl_2F$ | $n_D^{23}$: 1.5022 |
| $-C_2H_5$ | H | $CH_3$ | $-OCF_2CCl_2F$ | Sdp: 135-136°/ 0,02 mbar |
| $-CH_2OCH_3$ | H | $CH_3$ | $OCF_2CHFCF_3$ | $n_D^{23}$: 1.4668 |

| $R_1$ | $R_2$ | $R_c$ | $R_b$ | Physikalische Konstante |
|---|---|---|---|---|
| $-C_2H_5$ | H | $-OCHF_2$ | Br | Sdp: 113-115°/ 0,02 mbar |
| $CH_3$ | H | Cl | $-OCHF_2$ | $n_D^{24}$: 1.5234 |
| $-C_2H_5$ | H | Cl | $-OCHF_2$ | $n_D^{24}$: 1.5173 |
| $-CH_2OCH_3$ | H | $CH_3$ | $-OCF_2CCl_2F$ | $n_D^{23}$: 1.5012 |
| $-C_3H_7$-n | H | Cl | $-OCHF_2$ | $n_D^{22}$: 1.5126 |
| $-CH_2OCH_3$ | H | Cl | $-OCHF_2$ | Smp.: 75-78° |
| $-CH_2OCH_3$ | H | $CH_3$ | $-OCF_2CHCl_2$ | $n_D^{22}$: 1.5176 |
| $CH_3$ | H | $CH_3$ | $-OCF_2CHF_2$ | $n_D^{25}$: 1.4852 |
| $CH_3$ | $CH_3$ | $-OCHF_2$ | F | $n_D^{26}$: 1.4886 |
| $-C_2H_5$ | H | $-OCHF_2$ | $-OCHF_2$ | Sdp.: 116-117°/ 0,04 mbar |
| $-CH_2OCH_3$ | H | $-OCHF_2$ | Cl | Sdp.: 107-109°/ 0,05 mbar |
| $CH_3$ | $-C_2H_5$ | $CH_3$ | $-OCHF_2$ | $n_D^{23}$: 1.5030 |
| $CH_3$ | $-C_2H_5$ | $-OCHF_2$ | F | $n_D^{25}$: 1.4859 |
| $-C_2H_5$ | H | $-OCHF_2$ | Cl | Sdp.: 113-115°/ 0,05 mbar |
| $-CH_2OCH_2C{\equiv}CH$ | H | $-OCHF_2$ | Cl | $n_D^{27}$: 1.4931 |
| $-C_3H_7$-n | H | $-OCHF_2$ | $-OCHF_2$ | Sdp.: 125-126°/ 0,05 mbar |

| $R_1$ | $R_2$ | $R_c$ | $R_b$ | Physikalische Konstante |
|---|---|---|---|---|
| $-C_2H_5$ | H | $CH_3$ | $-OCHF_2$ | Sdp.: 105-108°/ 0,04 mbar |
| $CH_3$ | H | $-OCHF_2$ | F | $n_D^{26}$: 1.4931 |
| $-CH_2OCH_3$ | H | $-OCHF_2$ | F | $n_D^{26}$: 1.4918 |
| $CH_3$ | H | $-OCHF_2$ | Cl | $n_D^{24}$: 1.4976 |
| $-C_2H_5$ | H | $-OCHF_2$ | F | $n_D^{26}$: 1.4885 |
| $-C_3H_7$-n | H | $-OCHF_2$ | Cl | $n_D^{23}$: 1.4899 |
| $CH_3$ | $CH_3$ | $-OCHF_2$ | $-OCHF_2$ | Sdp.: 100-102°/ 0,04 mbar |
| $CH_3$ | $CH_3$ | $-OCHF_2$ | Cl | Sdp.: 107-112°/ 0,04 mbar |
| $-C_3H_7$-n | H | $-OCHF_2$ | F | $n_D^{26}$: 1.4881 |

Auf analoge Weise lassen sich auch die nachfolgenden Zwischenprodukte (falls nicht besonders vermerkt, sind es Diastereomerengemische mit unterschiedlichen Mischungsverhältnissen) der Formel III herstellen:

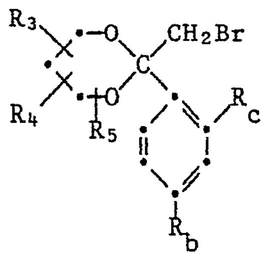

(III)

| $R_3$ | $R_4$ | $R_5$ | $R_c$ | $R_b$ | Physikalische Konstante |
|---|---|---|---|---|---|
| 4-$CH_3$ | H | H | -$OCHF_2$ | F | $n_D^{22}$: 1.5006 |
| 5-$CH_3$ | 5-$CH_3$ | H | -$OCHF_2$ | F | $n_D^{25}$: 1.4937 |
| 4-$CH_3$ | H | H | -$OCHF_2$ | Br | $n_D^{28}$: 1.5197 |
| 4-$CH_3$ | H | H | -$OCHF_2$ | Cl | Sdp.: 113-116°/ 0,09 mbar |
| 5-$CH_3$ | 5-$CH_3$ | H | -$OCHF_2$ | Br | $n_D^{26}$: 1.5136 |
| 4-$CH_3$ | H | H | -$OCHF_2$ | -$OCHF_2$ | Sdp.: 125-127°/ 0,07 mbar |
| 5-$CH_3$ | 5-$CH_3$ | H | -$OCHF_2$ | Cl | $n_D^{23}$: 1.4997 |
| 4-$CH_3$ | H | H | Cl | -$OCHF_2$ | $n_D^{22}$: 1.5089 |
| 4-$CH_3$ | H | H | $CH_3$ | -$OCHF_2$ | $n_D^{23}$: 1.5048 |
| H | H | H | Cl | -$OCHF_2$ | $n_D^{25}$: 1.4987 |
| 5-$CH_3$ | 5-$CH_3$ | H | Cl | -$OCHF_2$ | $n_D^{22}$: 1.5173 |
| 5-$CH_3$ | 5-$CH_3$ | H | $CH_3$ | -$OCHF_2$ | $n_D^{24}$: 1.5097 |
| 4-$CH_3$ | H | H | Br | -$OCHF_2$ | Sdp.: 109-111°/ 0,05 mbar |

Nach Art der Beispiele 1.1 oder 1.2 oder nach Art einer der weiter oben gegebenen Methoden lassen sich folgende Verbindungen der Formel I herstellen (falls nicht besonders vermerkt, sind es Diastereomerengemische mit unterschiedlichen Mischungsverhältnissen):

Tabelle 1: Verbindungen der Formel

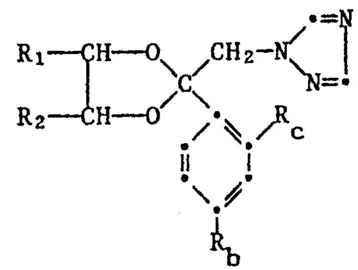

| Verb. Nr. | $R_1$ | $R_2$ | $R_c$ | $R_b$ | Salz | Physikalische Konstante |
|---|---|---|---|---|---|---|
| 1.1 | $CH_3$ | H | $CH_3$ | $-OCF_2CHF_2$ | --- | $n_D^{24}$:1.4868 |
| 1.2 | $-C_3H_7$-n | H | $-OCHF_2$ | $-OCHF_2$ | --- | $n_D^{23}$: 1.4841 |
| 1.3 | $-C_2H_5$ | H | $-OCHF_2$ | Cl | --- | $n_D^{27}$: 1.5132 |
| 1.4 | $-C_2H_5$ | H | $CH_3$ | $-OCHF_2$ | --- | $n_D^{28}$: 1.5067 |
| 1.5 | $-C_2H_5$ | H | $CH_3$ | $-OCF_2CFCl_2$ | HCl | |
| 1.6 | $-C_2H_5$ | H | $-OCHF_2$ | $-OCHF_2$ | --- | $n_D^{23}$: 1.4860 |

Tabelle 1 (Fortsetzung)

| Verb. Nr. | $R_1$ | $R_2$ | $R_c$ | $R_b$ | Salz | Physikalische Konstante |
|---|---|---|---|---|---|---|
| 1.7 | $CH_3$ | H | $CH_3$ | $-OCF_2CHFCl$ | --- | $n_D^{25}$: 1.4970 |
| 1.8 | $CH_3$ | $CH_3$ | $-OCHF_2$ | $-OCHF_2$ | --- | $n_D^{28}$: 1.4856 |
| 1.9 | $-CH_2OCH_3$ | H | $-OCHF_2$ | Cl | --- | $n_D^{22}$: 1.5164 |
| 1.10 | $CH_3$ | H | $-OCHF_2$ | F | --- | |
| 1.11 | $-C_2H_5$ | H | $CH_3$ | $-OCF_2CCl_3$ | • 1/2 $CaCl_2$ | |
| 1.12 | $-C_3H_7$-n | H | $-OCHF_2$ | Cl | --- | $n_D^{22}$: 1.5113 |
| 1.13 | $CH_3$ | $CH_3$ | $-OCHF_2$ | Cl | --- | $n_D^{22}$: 1.5150 |
| 1.14 | $CH_3$ | H | $CH_3$ | $-OCF_2CHCl_2$ | $HNO_3$ | |
| 1.15 | $-C_3H_7$-n | H | $-OCHF_2$ | F | --- | $n_D^{23}$: 1.4901 |

Tabelle 1 (Fortsetzung):

| Verb. Nr. | $R_1$ | $R_2$ | $R_c$ | $R_b$ | Salz | Physikalische Konstante |
|---|---|---|---|---|---|---|
| 1.16 | $CH_3$ | $CH_3$ | $CH_3$ | $-OCHF_2$ | --- | $n_D^{23}$: 1.5070 |
| 1.17 | $-C_2H_5$ | H | Cl | $-OCF_2CHFBr$ | --- | |
| 1.18 | $-CH_2OCH_3$ | H | $-OCHF_2$ | F | --- | |
| 1.19 | $-C_3H_7-n$ | H | $CH_3$ | $-OCHF_2$ | --- | $n_D^{23}$: 1.5050 |
| 1.20 | $CH_3$ | H | Cl | $-OCH_2CF_3$ | --- | |
| 1.21 | $CH_3$ | H | $CH_3$ | $-OCHF_2$ | --- | $n_D^{23}$: 1.5116 |
| 1.22 | $-CH_2OC_2H_5$ | H | $-OCHF_2$ | F | • $HNO_3$ | |
| 1.23 | $CH_3$ | H | $-OCHF_2$ | Br | --- | $n_D^{26}$: 1.5292 |
| 1.24 | $-C_2H_5$ | H | Cl | $-OCH_2CH_2Cl$ | --- | |
| 1.25 | $-CH_2OCH_2CF_3$ | H | $-OCHF_2$ | F | --- | $n_D^{25}$: 1.5137 |

Tabelle 1 (Fortsetzung):

| Verb. Nr. | $R_1$ | $R_2$ | $R_c$ | $R_b$ | Salz | Physikalische Konstante |
|---|---|---|---|---|---|---|
| 1.26 | 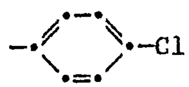 | H | $CH_3$ | $-OCHF_2$ | --- | |
| 1.27 | $-C_2H_5$ | H | $-OCHF_2$ | Br | --- | $n_D^{25}$: 1.5266 |
| 1.28 | $CH_3$ | H | Cl | $-OCH_2CH_2F$ | --- | |
| 1.29 | $-CH_2O-$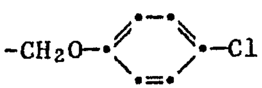 | H | $CH_3$ | $-OCHF_2$ | --- | |
| 1.30 | $-C_2H_5$ | H | Cl | $-OCH_2CCl_3$ | --- | |
| 1.31 | $-CH_2OCH_2CH=CH_2$ | H | $-OCHF_2$ | F | --- | |
| 1.32 | $-C_3H_7-n$ | H | $-OCHF_2$ | Br | | $n_D^{24}$: 1.5301 |
| 1.33 | $CH_3$ | H | Cl | $-OCCl_3$ | • 1/2 $CuCl_2$ | |
| 1.34 | $-CH_2OH$ | H | $CH_3$ | $-OCHF_2$ | --- | |

Tabelle 1 (Fortsetzung):

| Verb. Nr. | $R_1$ | $R_2$ | $R_c$ | $R_b$ | Salz | Physikalische Konstante |
|---|---|---|---|---|---|---|
| 1.35 | $-CH_2SC_2H_5$ | H | $CH_3$ | $-OCHF_2$ | --- | |
| 1.36 | $-CH_2OCH_2C{\equiv}CH$ | H | $CH_3$ | $-OCHF_2$ | --- | $n_D^{26}$: 1.5138 |
| 1.37 | $-C_2H_5$ | H | Cl | $-OCBr_3$ | --- | |
| 1.38 | $-C_3H_7$-i | H | $-OCHF_2$ | Br | --- | |
| 1.39 | $CH_3$ | H | Cl | $-OCF_2Br$ | --- | |
| 1.40 | $CH_3$ | $-C_2H_5$ | $-OCHF_2$ | F | --- | $n_D^{23}$: 1.5137 |
| 1.41 | $CH_3$ | $-C_2H_5$ | $CH_3$ | $-OCHF_2$ | --- | $n_D^{23}$: 1.5034 |
| 1.42 | $-C_2H_5$ | H | Cl | $-OC_2F_5$ | --- | |
| 1.43 | $-CH_2OCH_3$ | H | $CH_3$ | $-OCHF_2$ | --- | |
| 1.44 | $CH_3$ | H | F | $-OCHF_2$ | --- | |

Tabelle 1 (Fortsetzung):

| Verb. Nr. | $R_1$ | $R_2$ | $R_c$ | $R_b$ | Salz | Physikalische Konstante |
|---|---|---|---|---|---|---|
| 1.45 | H | H | Cl | $-OCHF_2$ | --- | |
| 1.46 | $CH_3$ | H | Cl | $-OCH_2Cl$ | --- | |
| 1.47 | H | H | $-OCHF_2$ | Cl | --- | |
| 1.48 | $CH_3$ | $-C_2H_5$ | $-OCHF_2$ | F | --- | |
| 1.49 | $-CH_2OCH_3$ | H | $-OCHF_2$ | Br | --- | |
| 1.50 | $-C_2H_5$ | H | Cl | $-OCHFCl$ | --- | |
| 1.51 | $-CH_2OC_2H_5$ | H | $-OCHF_2$ | Br | --- | |
| 1.52 | $CH_3$ | H | $-OCHF_2$ | Cl | --- | $n_D^{26}$: 1.5180 |
| 1.53 | $CH_3$ | H | Cl | $-OCH_2Br$ | --- | |

Tabelle 1 (Fortsetzung):

| Verb. Nr. | $R_1$ | $R_2$ | $R_c$ | $R_b$ | Salz | Physikalische Konstante |
|---|---|---|---|---|---|---|
| 1.54 | $-C_6H_{13}$-n | H | $-OCHF_2$ | Cl | --- | |
| 1.55 | $-CH_2OCH_2CF_3$ | H | $-OCHF_2$ | Br | --- | $n_D^{26}$: 1.4982 |
| 1.56 | $-CH_2OC_2H_5$ | H | $CH_3$ | $-OCHF_2$ | --- | |
| 1.57 | $-CH_2SC_3H_7$-i | H | $CH_3$ | $-OCHF_2$ | --- | |
| 1.58 | $-C_2H_5$ | H | F | $-OCHF_2$ | --- | |
| 1.59 | $-CH_2Cl$ | H | $-OCHF_2$ | Cl | --- | $n_D^{22}$: 1.5336 |
| 1.60 | 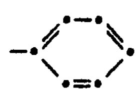 | H | $-OCHF_2$ | Cl | --- | $n_D^{23}$: 1.5570 |
| 1.61 | $-CH_2OCH_2CH=CH_2$ | H | $-OCHF_2$ | Br | --- | |
| 1.62 | $-C_3H_7$-n | H | F | $-OCHF_2$ | --- | |

Tabelle 1 (Fortsetzung):

| Verb. Nr. | $R_1$ | $R_2$ | $R_c$ | $R_b$ | Salz | Physikalische Konstante |
|---|---|---|---|---|---|---|
| 1.63 | $-CH_2OC_2H_5$ | H | $-OCHF_2$ | Cl | --- | |
| 1.64 | $-CH_2O(CH_2)_3OCH_3$ | H | $CH_3$ | $-OCHF_2$ | --- | |
| 1.65 | $CH_3$ | $-C_2H_5$ | $-OCHF_2$ | Br | --- | |
| 1.66 | $-CH_2OC_3H_7$-n | H | $CH_3$ | $-OCHF_2$ | --- | |
| 1.67 | $-CH_2OCH_3$ | H | F | $-OCHF_2$ | --- | |
| 1.68 | $CH_3$ | H | Cl | $-OCHF_2$ | --- | $n_D^{28}$:1,5208 |
| 1.69 | $-CH_2OCH_2CF_3$ | H | $-OCHF_2$ | Cl | --- | $n_D^{24}$: 1.4876 |
| 1.70 | $-CH_2OCH_3$ | H | $-OCHF_2$ | $-OCHF_2$ | --- | |
| 1.71 | $-CH_2OC_2H_5$ | H | F | $-OCHF_2$ | --- | |
| 1.72 | $CH_3$ | $CH_3$ | $-OCHF_2$ | Br | --- | $n_D^{26}$: 1.5268 |

<u>Tabelle 1</u> (Fortsetzung):

| Verb. Nr. | $R_1$ | $R_2$ | $R_c$ | $R_b$ | Salz | Physikalische Konstante |
|---|---|---|---|---|---|---|
| 1.73 | $-CH_2OC_3H_7-i$ | H | $CH_3$ | $-OCHF_2$ | --- | |
| 1.74 | $-CH_2OCH_2CF_3$ | H | F | $-OCHF_2$ | --- | |
| 1.75 | $-CH_2OCH_2CH=CH_2$ | H | $-OCHF_2$ | Cl | --- | |
| 1.76 | $-CH_2OC_2H_5$ | H | $-OCHF_2$ | $-OCHF_2$ | --- | |
| 1.77 | $-C_2H_5$ | H | Cl | $-OCHF_2$ | --- | $n_D^{29}$:1.5159 |
| 1.78 | $-CH_2OCH_2CF_3$ | H | $-OCHF_2$ | $-OCHF_2$ | --- | |
| 1.79 | $-CH_2OCH_2-C\equiv CH$ | H | $-OCHF_2$ | Cl | --- | |
| 1.80 | $CH_3$ | $-C_2H_5$ | F | $-OCHF_2$ | --- | |
| 1.81 | $-C_3H_7-n$ | H | Cl | $-OCHF_2$ | --- | $n_D^{28}$:1.5123 |

Tabelle 1 (Fortsetzung):

| Verb. Nr. | $R_1$ | $R_2$ | $R_c$ | $R_b$ | Salz | Physikalische Konstante |
|---|---|---|---|---|---|---|
| 1.82 | $-CH_2OC_6H_{13}$-n | H | $CH_3$ | $-OCHF_2$ | --- | |
| 1.83 | $-C_3H_7$-i | H | Cl | $-OCHF_2$ | --- | |
| 1.84 | $CH_3$ | $-C_2H_5$ | $-OCHF_2$ | Cl | --- | |
| 1.85 | $-CH_2OCH_2CH=CH_2$ | H | $-OCHF_2$ | $-OCHF_2$ | --- | |
| 1.86 | $CH_3$ | $-C_2H_5$ | $-OCHF_2$ | $-OCHF_2$ | --- | |
| 1.87 | $-CH_2OCH_2CF_3$ | H | $CH_3$ | $-OCHF_2$ | --- | |
| 1.88 | $-C_3H_7$-i | H | $CH_3$ | $-OCHF_2$ | --- | |
| 1.89 | $CH_3$ | $CH_3$ | F | $-OCHF_2$ | --- | |
| 1.90 | $-CH_2OCH_3$ | H | Cl | $-OCHF_2$ | --- | $n_D^{27}$:1.5185 |

Tabelle 1 (Fortsetzung):

| Verb. Nr. | $R_1$ | $R_2$ | $R_c$ | $R_b$ | Salz | Physikalische Konstante |
|---|---|---|---|---|---|---|
| 1.91 | $-CH_2OCH_2CH=CH_2$ | H | $CH_3$ | $-OCHF_2$ | --- | |
| 1.92 | $-C_3H_{13}$-n | H | $CH_3$ | $-OCHF_2$ | --- | |
| 1.93 | $-CH_2OC_2H_5$ | H | Cl | $-OCHF_2$ | --- | |
| 1.94 | $-CH_2OCH_2C\equiv CH$ | H | $CH_3$ | $-OCHF_2$ | --- | |
| 1.95 | $-CH_2OCH_2CF_3$ | H | Cl | $-OCHF_2$ | --- | |
| 1.96 | $-C_3H_7$-i | H | Br | $-OCHF_2$ | --- | |
| 1.97 | $-CH_2Cl$ | H | $CH_3$ | $-OCHF_2$ | --- | $n_D^{25}$: 1.4936 |
| 1.98 | $-CH_2OCH_3$ | H | Br | $-OCHF_2$ | --- | |
| 1.99 | $-CH_2O-$ 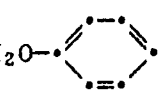 | H | $CH_3$ | $-OCHF_2$ | --- | |

Tabelle 1 (Fortsetzung):

| Verb. Nr. | $R_1$ | $R_2$ | $R_c$ | $R_b$ | Salz | Physikalische Konstante |
|---|---|---|---|---|---|---|
| 1.100 | 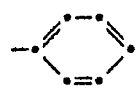 | H | $CH_3$ | $-OCHF_2$ | --- | |
| 1.101 | $-CH_2OC_2H_5$ | H | Br | $-OCHF_2$ | --- | |
| 1.102 | $-CH_2OCH_2CH{=}CH_2$ | H | Cl | $-OCHF_2$ | --- | |
| 1.103 | $CH_3$ | $-C_2H_5$ | Br | $-OCHF_2$ | --- | |
| 1.104 | $-C_3H_7$-n | H | Cl | $-OCF_2CFCl_2$ | --- | $n_D^{25}$: 1.5139 |
| 1.105 | $-CH_2OCH_2C{\equiv}CH$ | H | Br | $-OCHF_2$ | --- | |
| 1.106 | $-CH_2OCH_2C{\equiv}CH$ | H | Cl | $-OCHF_2$ | --- | |
| 1.107 | $CH_3$ | $CH_3$ | Br | $-OCHF_2$ | --- | |
| 1.108 | $CH_3$ | $-C_2H_5$ | Cl | $-OCHF_2$ | --- | |

Tabelle 1 (Fortsetzung):

| Verb. Nr. | $R_1$ | $R_2$ | $R_c$ | $R_b$ | Salz | Physikalische Konstante |
|---|---|---|---|---|---|---|
| 1.109 | $-CH_2OCH_3$ | H | Cl | $-OCF_2CFCl_2$ | --- | |
| 1.110 | $CH_3$ | $CH_3$ | Cl | $-OCHF_2$ | --- | |
| 1.111 | $-C_3H_7$-n | H | Br | $-OCHF_2$ | --- | |
| 1.112 | H | H | Br | $-OCHF_2$ | --- | |
| 1.113 | $-CH_2OCH_2CF_3$ | H | Br | $-OCHF_2$ | --- | |
| 1.114 | $-C_3H_7$-n | H | $CH_3$ | $-OCF_2CFCl_2$ | --- | $n_D^{23}$: 1.4981 |
| 1.115 | $CH_3$ | H | Br | $-OCHF_2$ | --- | |
| 1.116 | $-CH_2OCH_3$ | H | $CH_3$ | $-OCF_2CFCl_2$ | --- | $n_D^{23}$: 1.5025 |
| 1.117 | $-C_2H_5$ | H | Br | $-OCHF_2$ | --- | |

<u>Tabelle 1</u> (Fortsetzung):

| Verb. Nr. | $R_1$ | $R_2$ | $R_c$ | $R_b$ | Salz | Physikalische Konstante |
|---|---|---|---|---|---|---|
| 1.118 | $-C_3H_7$-n | H | $CH_3$ | $-OCF_2CHF_2$ | --- | $n_D^{25}$:1.4816 |
| 1.119 | $-CH_2OCH_3$ | H | $CH_3$ | $-OCF_2CHClF$ | --- | $n_D^{23}$:1.5047 |
| 1.120 | $-C_2H_5$ | H | $CH_3$ | $-OCF_2CHF_2$ | --- | $n_D^{24}$:1.4833 |
| 1.121 | $-C_2H_5$ | H | $CH_3$ | $-OCF_2CHClF$ | --- | $n_D^{24}$:1.4974 |
| 1.122 | $-CH_2OCH_3$ | H | $CH_3$ | $-OCF_2CHF_2$ | --- | $n_D^{25}$:1.4868 |
| 1.123 | $-C_3H_7$-n | H | $CH_3$ | $-OCF_2CHClF$ | --- | $n_D^{24}$:1.4955 |
| 1.124 | $-CH_2F$ | H | $-OCHF_2$ | Cl | --- | $n_D^{24}$:1.5397 |
| 1.125 | 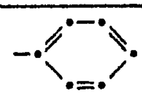 | H | $-OCHF_2$ | Br | --- | $n_D^{26}$:1.5661 |
| 1.126 | $-CH_2F$ | H | Cl | $-OCHF_2$ | --- | $n_D^{23}$:1.5312 |

Tabelle 1 (Fortsetzung):

| Verb. Nr. | $R_1$ | $R_2$ | $R_c$ | $R_b$ | Salz | Physikalische Konstante |
|---|---|---|---|---|---|---|
| 1.127 | $-CH_2Cl$ | H | Cl | $-OCHF_2$ | --- | $n_D^{22}$:1.5297 |
| 1.128 | $CH_3$ | H | $CH_3$ | $-OCF_2CFCl_2$ | --- | $n_D^{23}$:1.5033 |
| 1.129 | $-CH_2F$ | H | $CH_3$ | $-OCHF_2$ | --- | $n_D^{24}$:1.5288 |
| 1.130 | $-CH_2OC_4H_9$-sek. | H | $-OCHF_2$ | Cl | --- | $n_D^{24}$:1.5035 |
| 1.131 | $-C_2H_5$ | H | $CH_3$ | $-OCF_2CFCl_2$ | --- | $n_D^{23}$:1.5006 |
| 1.132 | $-CH_2OCH_2C\equiv CH$ | H | $-OCHF_2$ | Cl | --- | $n_D^{23}$:1.4983 |
| 1.133 | $-CH_2Cl$ | H | $-OCHF_2$ | Br | --- | $n_D^{22}$:1.5372 |
| 1.134 | $-CH_2F$ | H | $-OCHF_2$ | Br | --- | $n_D^{24}$:1.5293 |

Tabelle 2 Verbindungen der Formel

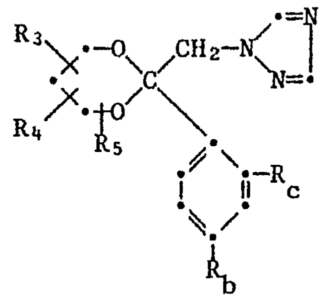

| Verb. Nr. | $R_3$ | $R_4$ | $R_5$ | $R_c$ | $R_b$ | Salz | Physikalische Konstante |
|---|---|---|---|---|---|---|---|
| 2.1 | 4-$CH_3$ | H | H | $OCHF_2$ | F | $HNO_3$ | |
| 2.2 | 4-$CH_3$ | H | H | $OCHF_2$ | Cl | --- | $n_D^{24}$:1,4973 |
| 2.3 | 5-$C_2H_5$ | 5-$C_2H_5$ | H | $CH_3$ | -$OCHF_2$ | --- | |
| 2.4 | 5-$CH_3$ | 5-$CH_3$ | H | -$OCHF_2$ | F | --- | $n_D^{23}$:1.5134 |
| 2.5 | 4-$CH_3$ | 5-$CH_3$ | 6-$CH_3$ | Cl | -$OCHF_2$ | --- | |
| 2.6 | 4-$CH_3$ | H | H | $CH_3$ | -$OCF_2CHF_2$ | --- | |

Tabelle 2 (Fortsetzung):

| Verb. Nr. | $R_3$ | $R_4$ | $R_5$ | $R_c$ | $R_b$ | Salz | Physikalische Konstante |
|---|---|---|---|---|---|---|---|
| 2.7 | 5-$CH_3$ | 5-$CH_3$ | H | $CH_3$ | -$OCF_2CHF_2$ | 1/2 $CuCl_2$ | |
| 2.8 | H | H | H | -$OCHF_2$ | Cl | --- | |
| 2.9 | 5-$CH_3$ | 5-$C_3H_7$-n | H | $CH_3$ | -$OCHF_2$ | --- | |
| 2.10 | 4-$CH_3$ | H | H | Cl | -$OCF_2CCl_3$ | $HNO_3$ | |
| 2.11 | 5-$CH_3$ | 5-$CH_3$ | H | -$OCHF_2$ | Cl | --- | $n_D^{22}$:1.5146 |
| 2.12 | 4-$CH_3$ | 6-$C_3H_7$-n | H | $CH_3$ | -$OCHF_2$ | --- | |
| 2.13 | 5-$CH_3$ | 5-$CH_3$ | H | Cl | -$OCF_2CCl_3$ | --- | |
| 2.14 | 4-$CH_3$ | H | H | -$OCHF_2$ | Br | --- | $n_D^{24}$:1.5234 |
| 2.15 | 4-$CH_3$ | H | H | F | -$OCHF_2$ | --- | |

Tabelle 2 (Fortsetzung)

| Verb. Nr. | $R_3$ | $R_4$ | $R_5$ | $R_c$ | $R_b$ | Salz | Physikalische Konstante |
|---|---|---|---|---|---|---|---|
| 2.16 | H | H | H | Cl | $-OCF_2CFCl_2$ | --- | |
| 2.17 | 5-$CH_3$ | 5-$CH_3$ | H | $-OCHF_2$ | Br | --- | $n_D^{23}$:1.5297 |
| 2.18 | 4-$CH_3$ | H | H | Cl | $-OCF_2CFCl_2$ | --- | |
| 2.19 | H | H | H | $CH_3$ | $-OCF_3$ | --- | |
| 2.20 | 5-$CH_3$ | 5-$CH_3$ | H | F | $-OCHF_2$ | --- | |
| 2.21 | 4-$CH_3$ | H | H | $-OCHF_2$ | $-OCHF_2$ | --- | $n_D^{25}$:1.5173 |
| 2.22 | 5-$CH_3$ | 5-$CH_3$ | H | Cl | $-OCF_2CFCl_2$ | --- | |
| 2.23 | H | H | H | Cl | $-OCHF_2$ | --- | $n_D^{24}$:1.4912 |
| 2.24 | 5-$CH_3$ | 5-$CH_3$ | H | $-OCHF_2$ | $-OCHF_2$ | --- | |

Tabelle 2 (Fortsetzung)

| Verb. Nr. | $R_3$ | $R_4$ | $R_5$ | $R_c$ | $R_b$ | Salz | Physikalische Konstante |
|---|---|---|---|---|---|---|---|
| 2.25 | 5-$CH_3$ | 5-$CH_3$ | H | Br | -$OCHF_2$ | --- | |
| 2.26 | 5-$CH_3$ | 5-$C_2H_5$ | H | Cl | -$OCF_2CFCl_2$ | --- | |
| 2.27 | H | H | H | $CH_3$ | -$OCHF_2$ | --- | |
| 2.28 | 4-$CH_3$ | H | H | Cl | -$OCHF_2$ | --- | $n_D^{24}$:1.4913 |
| 2.29 | 4-$CH_3$ | H | H | Cl | -$OCF_3$ | --- | |
| 2.30 | 4-$CH_3$ | H | H | $CH_3$ | -$OCHF_2$ | --- | $n_D^{25}$:1.4897 |
| 2.31 | 4-$C_2H_5$ | H | H | $CH_3$ | -$OCHF_2$ | --- | |
| 2.32 | 4-$CH_3$ | 4-$CH_3$ | H | Cl | -$OCHF_2$ | --- | |
| 2.33 | 5-$CH_3$ | 5-$CH_3$ | H | Cl | -$OCF_3$ | --- | $n_D^{23}$:1.5092 |

<u>Tabelle 2</u> (Fortsetzung)

| Verb. Nr. | $R_3$ | $R_4$ | $R_5$ | $R_c$ | $R_b$ | Salz | Physikalische Konstante |
|---|---|---|---|---|---|---|---|
| 2.34 | $5-CH_3$ | $5-CH_3$ | H | $CH_3$ | $-OCHF_2$ | --- | $n_D^{25}$:1.5176 |
| 2.35 | $5-CH_3$ | $5-C_2H_5$ | H | $CH_3$ | $-OCF_3$ | --- | |
| 2.36 | $5-CH_3$ | $5-C_2H_5$ | H | Cl | $-OCHF_2$ | --- | |
| 2.37 | $5-CH_3$ | $5-C_2H_5$ | H | $CH_3$ | $-OCHF_2$ | --- | |
| 2.38 | $4-CH_3$ | H | H | Br | $-OCHF_2$ | --- | $n_D^{23}$:1.5096 |
| 2.39 | $5-CH_3$ | $5-CH_3$ | H | Cl | $-OCHF_2$ | --- | $n_D^{23}$:1.5072 |

Tabelle 3: Verbindungen der Formel

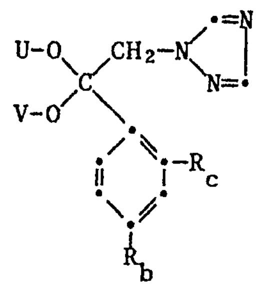

| Verb. Nr. | U | V | $R_c$ | $R_b$ | Salz | Physikalische Konstante |
|---|---|---|---|---|---|---|
| 3.1 | $CH_3$ | $CH_3$ | $-OCHF_2$ | F | --- | |
| 3.2 | $CH_3$ | $CH_3$ | $CH_3$ | $-OCHF_2$ | --- | $n_D^{23}$:1.5072 |
| 3.3 | $C_2H_5$ | $C_2H_5$ | $-OCHF_2$ | Cl | --- | $n_D^{22}$:1.5132 |
| 3.4 | $C_4H_9$ sek. | $C_4H_9$ sek. | $CH_3$ | $-OCHF_2$ | --- | |
| 3.5 | $C_3H_7$-n | $C_3H_7$-n | $-OCHF_2$ | Cl | --- | |
| 3.6 | $C_{12}H_{25}$-n | $C_{12}H_{25}$-n | $CH_3$ | $-OCHF_2$ | --- | |
| 3.7 | $CH_3$ | $CH_3$ | $-OCHF_2$ | Br | --- | $n_D^{25}$:1.5176 |

Tabelle 3 (Fortsetzung)

| Verb. Nr. | U | V | $R_c$ | $R_b$ | Salz | Physikalische Konstante |
|---|---|---|---|---|---|---|
| 3.8 | $C_4H_9$-n | $C_4H_9$-n | $CH_3$ | $-OCHF_2$ | --- | |
| 3.9 | $-CH_2CF_3$ | $-CH_2CF_3$ | $-OCHF_2$ | $-OCHF_2$ | --- | |
| 3.10 | $-CH_2CH_2Cl$ | $-CH_2CH_2Cl$ | $CH_3$ | $-OCHF_2$ | --- | |
| 3.11 | $CH_3$ | $CH_3$ | F | $-OCHF_2$ | --- | |
| 3.12 | $CH_3$ | $CH_3$ | Cl | $-OCHF_2$ | --- | |
| 3.13 | $C_2H_5$ | $C_2H_5$ | Br | $-OCHF_2$ | --- | |
| 3.14 | $C_3H_7$-i | $C_3H_7$-i | Cl | $-OCHF_2$ | --- | $n_D^{25}$:1.5112 |
| 3.15 | $C_3H_7$-i | $C_3H_7$-i | $-OCHF_2$ | Cl | --- | |
| 3.16 | $C_4H_9$sek | $C_4H_9$sek | Cl | $-OCHF_2$ | --- | |
| 3.17 | $C_4H_9$-i | $C_4H_9$-i | Cl | $-OCHF_2$ | --- | |

2. Formulierungsbeispiele für flüssige Wirkstoffe der Formel I (% = Gewichtsprozent)

| 2.1 Emulsions-Konzentrate | a) | b) | c) |
|---|---|---|---|
| Wirkstoff aus den Tabellen | 25 % | 40 % | 50 % |
| Ca-Dodecylbenzolsulfonat | 5 % | 8 % | 6 % |
| Ricinusöl-polyethylenglykolether (36 Mol Ethylenoxid) | 5 % | - | - |
| Tributylphenoyl-polyethylenglykol-ether (30 Mol Ethylenoxid) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2.2 Lösungen | a) | b) | c) | d) |
|---|---|---|---|---|
| Wirkstoff aus den Tabellen | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190°C) | - | - | 94 % | - |

(MG = Molekulargewicht)

Die Lösungen sind zur Anwendung in Form kleinster Tropfen geeignet.

| 2.3 Granulate | a) | b) |
|---|---|---|
| Wirkstoff aus den Tabellen | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 2.4 Stäubemittel | a) | b) |
|---|---|---|
| Wirkstoff aus den Tabellen | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Formulierungsbeispiele für feste Wirkstoffe der Formel I

(% = Gewichtsprozent)

| 2.5 Spritzpulver | a) | b) | c) |
|---|---|---|---|
| Wirkstoff aus den Tabellen | 25 % | 50 % | 75 % |
| Na-Ligninsulfonat | 5 % | 5 % | |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 2.6 Emulsions-Konzentrat | |
|---|---|
| Wirkstoff aus den Tabellen | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 2.7 Stäubemittel | a) | b) |
|---|---|---|
| Wirkstoff aus den Tabellen | 5 % | 8 % |
| Talkum | 95 % | - |
| Kaolin | - | 92 % |

Man erhält anwendungsfertige Stäubemittel, indem der Wirkstoff mit den Träger vermischt und auf einer geeigneten Mühle vermahlen wird.

| 2.8 Extruder Granulat | |
|---|---|
| Wirkstoff aus den Tabellen | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| 2.9 Umhüllungs-Granulat | |
|---|---|
| Wirkstoff aus den Tabellen | 3 % |
| Polyethylenglykol (MG 200) | 3 % |
| Kaolin | 94 % |
| (MG = Molekulargewicht) | |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

| 2.10 Suspensions-Konzentrat | |
|---|---|
| Wirkstoff aus den Tabellen | 40 % |
| Ethylenglykol | 10 % |
| Nonylphenolpolyethylenglykolether (15 Mol Ethylenoxid) | 6 % |
| N-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

3. Biologische Beispiele

Beispiel 3.1: Wirkung gegen Puccinia graminis auf Weizen

a) Residual-protektive Wirkung

Weizenpflanzen werden 6 Tage nach der Aussaat mit einer aus Spritzpulver des Wirkstoffes hergestellten Spritzbrühe (0,02 % Aktivsubstanz) besprüht. Nach 24 Stunden werden die behandelten Pflanzen mit einer Uredosporensuspension des Pilzes infiziert. Nach einer Inkubation während 48 Stunden bei 95-100 % relativer Luftfeuchtigkeit und ca. 20° C werden die infizierten Pflanzen in einem Gewächshaus bei ca. 22° C aufgestellt. Die Beurteilung der Rostpustelnentwicklung erfolgt 12 Tage nach der Infektion.

b) Systemische Wirkung

Zu Weizenpflanzen wird 5 Tage nach der Aussaat eine aus Spritzpulver des Wirkstoffes hergestellte Spritzbrühe gegossen (0,006 % Aktivsubstanz bezogen auf das Bodenvolumen). Nach 48 Stunden werden die behandelten Pflanzen mit einer Uredosporensuspension des Pilzes infiziert. Nach einer Inkubation während 48 Stunden bei 95-100 % relativer Luftfeuchtigkeit und ca. 20° C werden die infizierten Pflanzen in einem Gewächshaus bei ca. 22° C aufgestellt. Die Beurteilung der Rostpustelnentwicklung erfolgt 12 Tage nach der Infektion.

Verbindungen aus den Tabellen zeigen gegen Puccinia-Pilze gute Wirksamkeit. Unbehandelte aber infizierte Kontrollpflanzen weisen dagegen einen Puccinia-Befall von 50-100 % auf. So reduzieren z.B. die Verbindungen Nr. 1.2; 1.3; 1.6; 1.8; 1.9; 1.12; 1.13; 1.15, 1.16, 1.19; 1.27; 1.52; 1.55; 1.59; 1.68; 1.69; 1.72; 1.81; 1.90; 1.118; 1.120; 1.122; 1.124; 1.125; 1.126; 1.127; 1.129; 1.131; 1.133; 1.134 und 2.2; 2.11; 2.14; 2.28; 2.39; 3.3; 3.4; 3.7; 3.14 den Puccinia-Befall fast vollständig (0-10 %).

Beispiel 3.2: Wirkung gegen Cercospora archidicola auf Erdnusspflanzen

Residual-protektive Wirkung

10-15 cm hohe Erdnusspflanzen werden mit einer aus Spritzpulver der Wirksubstanz hergestellten Spritzbrühe (0,02 % Aktivisubstanz) besprüht und 48 Stunden später mit einer Konidiensuspension des Pilzes infiziert. Die infizierten Pflanzen werden während 72 Stunden bei ca. 21° C und hoher Luftfeuchtigkeit inkubiert und anschliessend bis zum Auftreten der typischen Blattflecken in einem Gewächshaus aufgestellt. Die Beurteilung der fungiziden Wirkung erfolgt 12 Tage nach der Infektion basierend auf Anzahl und Grösse der auftretenden Flecken.

Im Vergleich zu unbehandelten, aber infizierten Kontrollpflanzen (Anzahl und Grösse der Flecken = 100 %), zeigten Erdnusspflanzen, die mit Wirkstoffen aus den Tabellen behandelt wurden, einen stark reduzierten Cercospora-Befall. So verhindern z.B. die Verbindungen Nr. 1.1;1.2; 1.3; 1.4; 1.6; 1.7; 1.9; 1.12; 1.13, 1.15; 1.16; 1.19; 1.21; 1.23; 1.27; 1.52; 1.55; 1.59; 1.68; 1.69; 1.72; 1.77; 1.81; 1.90; 1.116; 1.118; 1.119; 1.120; 1.121; 1.122; 1.123; 1.124; 1.125; 1.127; 1.128; 1.129; 1.131; 1.133; 1.134; 2.2; 2.11; 2.14; 2.30; 2.39 und 3.3; 3.7 im obigen Versuch das Auftreten von Flecken fast vollständig (0-10 %).

Beispiel 3.3: Wirkung gegen Erysiphae graminis auf Gerste

a) Residual-protektive Wirkung

Ca. 8 cm hohe Gerstenpflanzen werden mit einer aus Spritzpulver des Wirkstoffes hergestellten Spritzbrühe (0,02 % Aktivsubstanz) besprüht. Nach 3-4 Stunden werden die behandelten Pflanzen mit Konidien des Pilzes bestäubt. Die infizierten Gerstenpflanzen werden in einem Gewächshaus bei ca. 22° C aufgestellt und der Pilzbefall nach 10 Tagen beurteilt.

b) Systemische Wirkung

Zu ca. 8 cm hohen Gerstenpflanzen wird eine aus Spritzpulver des Wirkstoffes hergestellte Spritzbrühe gegossen (0,006 % Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden werden die behandelten Pflanzen mit Konidien des Pilzes bestäubt. Die infizierten Gerstenpflanzen werden in einem Gewächshaus bei ca. 22° C aufgestellt und der Pilzbefall nach 10 Tagen beurteilt.

Verbindungen aus den Tabellen zeigen gute Wirksamkeit gegen den Erysiphe-Pilz. Unbehandelte, aber infizierte Kontrollpflanzen weisen dagegen einen Erysiphe-Befall von 100 % auf. So hemmen z.B. die Verbindungen Nr. 1.1; 1.2; 1.3; 1.4; 1.6; 1.7; 1.8; 1.9; 1.12; 1.13; 1.15, 1.16; 1.19; 1.21; 1.23; 1.27; 1.52; 1.55; 1.59; 1.60; 1.68; 1.69; 1.72; 1.77; 1.81; 1.90; 1.118; 1.119; 1.120; 1.122; 1.123; 1.124; 1.125; 1.126; 1.127; 1.128; 1.129; 1.131; 1.133; 1.134; 2.2; 2.11; 2.14;; 2.28; 2.39 und 3.2; 3.3; 3.7; 3.14 den Pilzbefall bei Gerste auf 0 bis 5 %.

Beispiel 3.4: Residual-protektive Wirkung gegen Venturia inaequalis auf Apfeltrieben

Apfelstecklinge mit 10-20 cm langen Frischtrieben werden mit einer aus Spritzpulver des Wirkstoffes hergestellten Spritzbrühe (0,02 % Aktivsubstanz) besprüht. Nach 24 Stunden werden die behandelten Pflanzen mit einer Konidiensuspension des Pilzes infiziert. Die Pflanzen werden dann während 5 Tagen bei

90-100 % relativer Luftfeuchtigkeit inkubiert und während 10 weiteren Tagen in einem Gewächshaus bei 20-24° C aufgestellt. Der Schorfbefall wird 15 Tage nach der Infektion beurteilt. Verbindungen aus den Tabellen hemmen den Krankheitsbefall bis auf weniger als 10 %. Unbehandelte jedoch infizierte Triebe zeigen dagegen einen 100%igen Venturia-Befall. So hemmen z.B. die Verbindungen Nr. 1.1; 1.2; 1.3; 1.4; 1.6; 1.7; 1.9; 1.12; 1.15 und 1.16, 1.19; 1.21; 1.68; 1.77; 1.81; 1.90; 1.118; 1.122; 1.123; 2.2; 2.11; 2.39 und 3.3 den Pilzbefall auf Apfeltrieben auf 0 bis 5 %.

Beispiel 3.5: Wirkung gegen Botrytis cinerea auf Aepfeln

Residual protektive Wirkung

Künstlich verletzte Aepfel werden behandelt, indem eine aus Spritzpulver der Wirksubstanz hergestellte Spritzbrühe (0,02 % AS) auf die Verletzungsstellen aufgetropft wird. Die behandelten Früchte werden anschliessend mit einer Sporensuspension von Botrytis cinerea inokuliert und während einer Woche bei hoher Luftfeuchtigkeit und ca. 20° C inkubiert.

Bei der Auswertung werden die pilzbefallenen Verletzungsstellen gezählt und daraus die fungizide Wirkung der Testsubstanz abgeleitet.

So vermindern z.B. die Verbindungen Nr. 1.4; 1.12; 1.15; 1.16, 1.19; 1.27; 1.68; 1.77; 1.81 und 1.90 den Pilzbefall auf 0 bis 5 %.

Beispiel 3.6: Wirkung gegen Pyricularia oryzae auf Reispflanzen

a) Residual-protektive Wirkung

Reispflanzen werden nach zweiwöchiger Anzucht mit einer aus Spritzpulver des Wirkstoffes hergestellten Spritzbrühe (0,02 % Aktivsubstanz) besprüht. Nach 48 Stunden werden die behandelten Pflanzen mit einer Konidiensuspension des Pilzes infiziert. Nach 5 Tagen Inkubation bei 95-100 % relativer Luftfeuchtigkeit und 24° C wird der Pilzbefall beurteilt.

b) Systemische Wirkung

Zu zwei Wochen alten in für Blumen üblichen Tontöpfen wachsenden Reispflanzen wird eine aus Spritzpulver des Wirkstoffes hergestellte Spritzbrühe gegossen (0,006 % Aktivsubstanz bezogen auf das Erdvolumen). Darauf werden die Töpfe mit Wasser soweit gefüllt, dass die untersten Stengelteile der Reispflanzen im Wasser stehen. Nach 48 Stunden werden die behandelten Reispflanzen mit einer Konidiensuspension des Pilzes infiziert. Nach einer Inkubation der infizierten Pflanzen während 5 Tagen bei 95-100 % relativer Luftfeuchtigkeit und ca. 24° wird der Pilzbefall beurteilt.

Verbindungen aus den Tabellen zeigen gute Wirksamkeit gegen den Pyriculariapilz. Unbehandelte, jedoch infizierte Kontrollpflanzen weisen dagegen einen Pyricularia-Befall von 100 % auf. So hemmen z.B. die Verbindungen 1.3; 1.15 und 1.52; 1.90; 1.119 den Pilzbefall bis auf 0 bis 5 %.

Beispiel 3.7: Wirkung gegen Tilletia caries an Weizen

Künstlich mit Brandsporen von Tilletia caries infizierte Wintergerste der Sorte Probus (3 g trockenes Sporenmaterial auf 1 kg Saatgut) wird auf einer Mischrolle mit dem zu prüfenden Fungizid gebeizt, wobei eine Konzentration von 60 ppm AS (bezogen auf das Gewicht des Saatgutes) zur Anwendung gelangt.

Der infizierte und behandelte Weizen wird im Oktober im Freiland mittels einer Sämaschine auf Parzellen von 2 m Länge und 3 Saatreihen mit dreifacher Wiederholung ausgesät.

Zur Ermittlung der Wirkstoffaktivität werden zum Zeitpunkt der Aehrenreife der prozentuale Anteil Tilletia-befallener Aehren ausgezählt.

Verbindungen aus den Tabellen zeigen gute Wirksamkeit gegen Tilletia. Unbehandelte jedoch infizierte Kontrollpflanzen weisen dagegen einen Tilletia-Befall von 100 % auf. So hemmen z.B. die Verbindungen 1.3; 1.16; 2.2 und 3.14 den Pilzbefall bis auf 0 bis 5 %.

Beispiel 3.8: Wirkung gegen Helminthosporium gramineum an Gerste

Auf natürliche Weise mit Helminthosporium gramineum infizierte Wintergerste der Sorte "Cl" wird auf einer Mischrolle mit dem zu prüfenden Fungizid gebeizt, wobei eine Konzentration von 60 ppm AS (bezogen auf das Gewicht des Saatgutes) zur Anwendung gelangt.

Die infizierte und behandelte Gerste wird im Oktober im Freiland mittels einer Sämaschine auf Parzellen von 2 m Länge und 3 Saatreihen mit dreifacher Wiederholung ausgesät.

Bis zur Befallsauswertung wird die Versuchspflanzung unter normalen Feldbedingungen kultiviert.

Zur Ermittlung der Wirkstoffaktivität werden zum Zeitpunkt des Aehrenschiebens der prozentuale Anteil Helminthosporium-befallener Halme ausgezählt.

Verbindungen aus den Tabellen zeigen gute Wirksamkeit gegen Helminthosporium. Unbehandelte jedoch infizierte Kontrollpflanzen weisen dagegen einen Helminthosporium-Befall von 100 % auf. So hemmen z.B. die Verbindungen 1.3 und 1.16 den Pilzbefall bis auf 0 bis 5 %.

Gemäss den in den vorausgehend beschriebenen biologischen Beispielen durchgeführten Tests wurden folgende strukturell nächststehende Verbindungen aus dem Stand der Technik im Vergleich mit Verbindungen der vorliegenden Erfindung geprüft.

Verbindungen aus dem Stand der Technik der Formel:

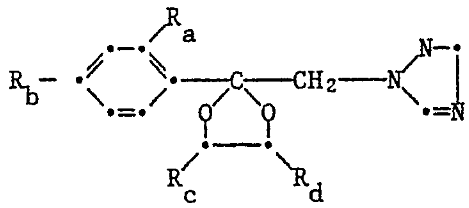

| Verb. | $R_a$ | $R_b$ | $R_c$ | $R_d$ | Publikation |
|---|---|---|---|---|---|
| A | H | H | H | H | US-4,160,838; Verb. 2/Tab. I |
| B | H | $OCH_3$ | H | H | US-4,160,838; Example XIV |
| C | H | $OCH_3$ | H | $CH_3$ | US-4,160,838; Example XX/5 |
| D | Cl | $OCH_3$ | H | H | US-4,160,838; Verb. 31/Tab. I |
| E | $OCH_3$ | H | H | H | US-4,160,838; Verb. 17/Tab. I |
| F | $OCH_3$ | Cl | H | H | US-4,160,838; Example XXXIII/6 |
| G | 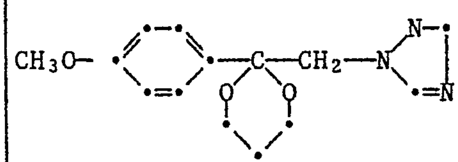 | | | | US-4,160,838; Example XXI/5 |

Biologische Tests (Residuale Wirkung)

1. Bewertungsskala

| Note | %-Aktivität | Pilzbefall |
|---|---|---|
| 1 | ≥ 95 | 0- 5 % |
| 3 | 80-95 | 5-20 % |
| 6 | 50-80 | 20-50 % |
| 9 | ≤ 50 | ≥50 % |

Eine Verbindung gilt als unwirksam, wenn der Pilzbefall an der Pflanze 50 % oder mehr beträgt.

2. Testergebnisse

| Verb. | Puccinia 200/20 ppm | | Cercospora 200/20 ppm | | Erysiphe 200/20 ppm | | Venturia 200/20 ppm | |
|---|---|---|---|---|---|---|---|---|
| A | 9 | 9 | 9 | 9 | 9 | 9 | 9 | 9 |
| B | 9 | 9 | 9 | 9 | 9 | 9 | 9 | 9 |
| C | 3 | 9 | 9 | 9 | 6 | 9 | 9 | 9 |
| D | 1 | 9 | 9 | 9 | 1 | 6 | 6 | 9 |
| E | 9 | 9 | 9 | 9 | 1 | 6 | 5 | 9 |
| F | 6 | 9 | 9 | 9 | 1 | 1 | 9 | 9 |
| G | 5 | 9 | 6 | 9 | 1 | 9 | 9 | 9 |
| 1.2 | 1 | 1 | 1 | 1 | 1 | 1 | 1 | 6 |
| 1.3 | 1 | 1 | 1 | 1 | 1 | 1 | 1 | 3 |
| 1.4 | 1 | 3 | 1 | 3 | 1 | 1 | 1 | 3 |
| 1.6 | 1 | 1 | 1 | 1 | 1 | 6 | 1 | 1 |
| 1.8 | 1 | 1 | 3 | 3 | 1 | 1 | 9 | 9 |
| 1.9 | 1 | 1 | 1 | 5 | 1 | 1 | 1 | 1 |
| 1.12 | 1 | 1 | 1 | 1 | 1 | 1 | 1 | 1 |
| 1.13 | 1 | 1 | 1 | 1 | 1 | 1 | 1 | 3 |
| 1.15 | 1 | 1 | 1 | 1 | 1 | 1 | 1 | 1 |
| 1.16 | 1 | 1 | 1 | 1 | 1 | 1 | 1 | 1 |
| 2.2 | 1 | 1 | 3 | 3 | 1 | 1 | 3 | 9 |
| 2.39 | 1 | 1 | 3 | 3 | 1 | 1 | 3 | 9 |
| 3.3 | 1 | 3 | 1 | 3 | 1 | 1 | 1 | 3 |

**Patentansprüche**

1. Verbindungen der Formel I

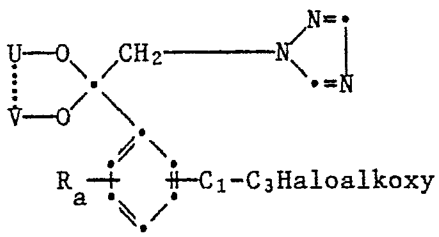

(I)

in welcher einer der beiden Phenylsubstituenten in 2-Stellung und der andere in 4-Stellung steht, und worin
$R_a$ für Halogen, Methyl oder $C_1$-$C_3$-Haloalkoxy bedeutet, und worin
U und V unabhängig voneinander gegebenenfalls durch Halogen oder $C_1$-$C_6$-Alkoxy substituiertes $C_1$-$C_{12}$-Alkyl darstellen oder zusammen eine der folgenden Alkylenbrücken

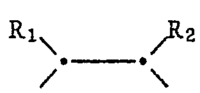 oder 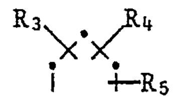

bilden wobei $R_1$ und $R_2$ unabhängig voneinander für Wasserstoff, $C_1$-$C_6$-Alkyl, ein- oder mehrfach durch Halogen substituiertes $C_1$-$C_6$-Alkyl, Phenyl, ein- oder mehrfach durch Halogen und/oder $C_1$-$C_2$-Alkyl substituiertes Phenyl oder die Gruppe -$CH_2$-Z-$R_6$ stehen oder worin $R_1$ und $R_2$ zusammen eine unsubstituierte oder durch $C_1$-$C_4$Alkyl substituierte Tetramethylenbrücke bilden; wobei
Z Sauerstoff oder Schwefel bedeutet;
$R_6$ für Wasserstoff, $C_1$-$C_6$-Alkyl, ein- oder mehrfach durch Halogen oder $C_1$-$C_3$-Alkoxy substituiertes $C_1$-$C_6$-Alkyl, oder für $C_3$-$C_4$-Alkenyl, 2-Propinyl, 3-Halo-2-propinyl, Phenyl oder Benzyl, deren aromatischer Ring unsubstituiert oder durch Halogen, $C_1$-$C_3$-Alkyl, $C_1$-$C_3$-Alkoxy, Nitro und/oder $CF_3$ ein- oder mehrfach substituiert ist, steht;
$R_3$, $R_4$ und $R_5$ unabhängig voneinander Wasserstoff oder $C_1$-$C_4$-Alkyl bedeuten, wobei die Gesamtzahl der Kohlenstoffatome in $R_3$, $R_4$ und $R_5$ die Zahl 6 nicht übersteigt;
unter Einschluss ihrer Säureadditionssalze und Metallsalzkomplexe.

2. Verbindungen der Formel I nach Anspruch 1, worin der Substituent "$C_1$-$C_3$ Haloalkoxy" mindestens eines oder aber mehrere gleiche oder verschiedene Halogenatome ausgewählt aus Fluor, Chlor und Brom enthält und, abhängig von den durch die Zahl der C-Atome gegebenen Möglichkeiten, 0-4 Wasserstoffatome besitzt;
$R_a$ für Fluor, Chlor, Brom, Methyl oder für ein beliebiges, wie vorstehend definiertes $C_1$-$C_3$ Haloalkoxy steht;
U und V unabhängig voneinander $C_1$-$C_6$ Alkyl darstellen oder zusammen eine der für Formel I genannten Alkylenbrücken bilden, worin
$R_1$ Wasserstoff oder $C_1$-$C_2$Alkyl, und
$R_2$ $C_1$-$C_6$ Alkyl, ein- oder mehrfach durch Halogen substituiertes $C_1$-$C_6$ Alkyl, Phenyl, ein- bis dreifach durch Halogen und/oder $C_1$-$C_2$ Alkyl substituiertes Phenyl oder die Gruppe -$CH_2$-O-$R_6$ stehen, bei der $R_6$ Wasserstoff, $C_1$-$C_6$ Alkyl, ein- oder mehrfach durch Halogen oder $C_1$-$C_3$ Alkoxy substituiertes $C_1$-$C_3$ Alkyl oder aber $C_3$-$C_4$ Alkenyl oder Propargyl darstellt, Phenyl oder Benzyl bedeutet, deren aromatischer Ring unsubstituiert oder durch Halogen, $C_1$-$C_2$ Alkyl, $C_1$-$C_2$ Alkoxy, Nitro und/oder $CF_3$ ein- bis dreifach substituiert ist; und worin $R_1$ und $R_2$ zusammen auch eine unsubstituierte oder methylsubstituierte Tetramethylenbrücke bilden können; und worin $R_3$, $R_4$ und $R_5$ unabhängig voneinander Wasserstoff oder Alkylgruppen mit insgesamt maximal 4 Kohlenstoffatomen bedeuten.

3. Verbindungen der Formel I nach Anspruch 2, bei denen die $C_1$-$C_3$ Haloalkoxygruppe eine der folgenden Bedeutungen hat:

A) $-OCHF_2$
B) $-OCF_2-CHF_2$
C) $-OCF_2-CFCl_2$
D) $-OCF_2-CHCl_2$
E) $-OCF_2-CHFCl$
F) $-OCF_2-CCl_3$
G) $-OCF_2-CFCl_2$
H) $-OCF_2-CHFBr$
I) $-OCH_2-CF_3$
J) $-OCH_2-CH_2Cl$
K) $-OCH_2-CH_2F$
L) $-OCH_2-CCl_3$
M) $-OCF_2-CHF-CF_3$
N) $-OCCl_3$
O) $-OCBr_3$
P) $-OCF_2Br$
Q) $-OC_2F_5$
R) $-OCF_3$
S) $-OCH_2Cl$
T) $-OCHFCl$
U) $-OCH_2Br$;

$R_a$ für Fluor, Chlor, Brom, Methyl oder für eine beliebige $C_1$-$C_3$ Haloalkoxygruppe vorstehender Bedeutung A) bis U) steht;
U und V unabhängig voneinander $C_1$-$C_6$ Alkyl darstellen oder zusammen eine der für Formel I genannten Alkylenbrücken bilden, worin
$R_1$ Wasserstoff oder $C_1$-$C_2$ Alkyl und
$R_2$ $C_1$-$C_6$ Alkyl, ein- oder mehrfach durch Fluor oder Chlor substituiertes $C_1$-$C_3$ Alkyl, Phenyl, ein- oder zweifach durch Halogen und/oder Methyl substituiertes Phenyl oder die Gruppe $-CH_2-O-R_6$ bedeuten, bei der
$R_6$ $C_1$-$C_6$ Alkyl, ein- oder mehrfach durch Fluor, und/oder Chlor substituiertes oder aber durch $C_1$-$C_3$-Alkoxy substituiertes $C_1$-$C_3$ Alkyl bedeutet oder aber $C_3$-$C_4$ Alkenyl, Propargyl oder Phenyl oder Benzyl ist, deren aromatischer Ring unsubstituiert oder durch Fluor, Chlor, Brom, Methyl, Methoxy, Nitro und/oder $CF_3$ ein- oder zweifach substituiert ist, und worin $R_1$ und $R_2$ zusammen auch eine unsubstituierte oder methylsubstituierte Tetramethylenbrücke bilden können; und worin $R_3$ Wasserstoff und $R_4$ und $R_5$ unabhängig voneinander Wasserstoff, Methyl, Aethyl oder n-Propyl bedeuten, zusammen jedoch 0 bis 4 Kohlenstoffatome besitzen.

4. Verbindungen der Formel I nach Anspruch 3, bei denen die $C_1$-$C_3$ Haloalkoxygruppe eine der folgenden Bedeutungen hat:
A) $-OCHF_2$ F) $-OCF_2-CCl_3$
B) $-OCF_2-CHF_2$ G) $-OCF_2-CFCl_2$
C) $-OCF_2-CFCl_2$ H) $-OCF_2-CHFBr$
D) $-OCF_2-CHCl_2$ I) $-OCH_2-CF_3$
E) $-OCF_2-CHFCl$ M) $-OCF_2-CHF-CF_3$;
$R_a$ für Fluor, Chlor, Brom, Methyl oder für eine beliebige $C_1$-$C_3$ Haloalkoxygruppe vorstehender Bedeutung A) bis I) oder M) steht.
U und V unabhängig voneinander $C_1$-$C_6$ Alkyl darstellen oder zusammen eine der für Formel I genannten Alkylenbrücken bilden, worin
$R_1$ Wasserstoff oder $C_1$-$C_2$ Alkyl und
$R_2$ $C_1$-$C_4$ Alkyl, ein- oder mehrfach durch Fluor oder Chlor substituiertes $C_1$-$C_2$ Alkyl, Phenyl, ein- oder zweifach durch Chlor und/oder Methyl substituiertes Phenyl oder die Gruppe $-CH_2-O-R_6$ bedeuten, bei der
$R_6$ $C_1$-$C_4$ Alkyl, ein- bis dreifach durch Fluor oder durch Methoxy substituiertes $C_1$-$C_3$ Alkyl bedeutet oder aber $C_3$-$C_4$ Alkenyl oder Propargyl oder aber Phenyl oder Benzyl ist, denen aromatischer Ring unsubstituiert oder durch Fluor, Chlor, Methyl, Methoxy, Nitro und/oder $CF_3$ ein- oder zweifach substituiert ist, und worin $R_1$ und $R_2$ zusammen auch eine unsubstituierte oder methylsubstituierte Tetramethylenbrücke bilden können; und worin
$R_3$ Wasserstoff
$R_4$ Wasserstoff, Methyl oder Aethyl, und
$R_5$ Wasserstoff, Methyl, Aethyl oder n-Propyl bedeuten, und $R_3$, $R_4$ und $R_5$ zusammen 0 bis 4 Kohlenstoffatome besitzen.

5. Verbindungen der Formel I nach Anspruch 4, bei denen die $C_1$-$C_3$-Haloalkoxygruppe eine der folgenden Bedeutungen hat:
A) $-OCHF_2$ E) $-OCF_2CHFCl$
B) $-OCF_2-CHF_2$ G) $-OCF_2CFCl_2$
$R_a$ für Fluor, Chlor, Brom, Methyl oder eine beliebige $C_1$-$C_2$ Haloalkoxygruppe vorstehender Bedeutung A), B), E) oder G) steht;
U und V unabhängig voneinander $C_1$-$C_6$-Alkyl darstellen oder zusammen eine der für Formel I genannten Alkylenbrücke bilden, worin
$R_1$ Wasserstoff oder $C_1$-$C_2$-Alkyl und
$R_2$ Wasserstoff, $C_1$-$C_4$-Alkyl, ein- oder mehrfach durch Fluor oder Chlor substituiertes $C_1$-$C_2$-Alkyl,

Phenyl, einfach durch Chlor und/oder Methyl substituiertes Phenyl oder die Gruppe $-CH_2OR_6$ bedeuten, bei der $R_6$ $C_1$-$C_4$-Alkyl, ein- bis dreifach durch Fluor oder durch Methoxy substituiertes $C_1$-$C_3$-Alkyl bedeutet oder aber $C_3$-$C_4$-Alkenyl oder Propargyl oder aber Phenyl ist, das unsubstituiert oder durch Fluor, Chlor, Methyl und/oder $CF_3$ monosubstituiert ist, und worin
$R_3$ Wasserstoff,
$R_4$ Wasserstoff, Methyl oder Aethyl und
$R_5$ Wasserstoff, Methyl oder Aethyl bedeuten, und
$R_3$, $R_4$ und $R_5$ zusammen 0 bis 4 Kohlenstoffatome besitzen.

6. Verbindungen der Formel I nach Anspruch 5, bei denen die Halogenalkoxygruppe
A) $-OCHF_2$ E) $-OCF_2CHFCl$
B) $-OCF_2CHF_2$ G) $-OCF_2CFCl_2$
bedeutet;
$R_a$ für Fluor, Chlor, Brom, Methyl, $OCHF_2$ oder $-OCF_2CHF_2$ steht;
U und V unabhängig voneinander $C_1$-$C_6$-Alkyl sind oder eine der in Formel I dargestellten Alkylenbrücken bilden, worin
$R_1$ Wasserstoff oder Methyl und
$R_2$ Wasserstoff, $C_1$-$C_3$-Alkyl, ein- oder mehrfach durch Fluor oder Chlor substituiertes $C_1$-$C_2$-Alkyl, Phenyl, einfach durch Chlor substituiertes Phenyl oder die Gruppe $-CH_2OR_6$ bedeuten, wobei $R_6$ $C_1$-$C_3$-Alkyl, ein bis dreifach durch Fluor substituiertes $C_1$-$C_2$-Alkyl oder $C_3$-$C_4$-Alkenyl oder Propargyl oder Phenyl darstellt, und worin
$R_3$ Wasserstoff, $R_4$ Wasserstoff, Methyl oder Aethyl und $R_5$ Wasserstoff oder Methyl bedeuten und $R_3$, $R_4$ und $R_5$ zusammen 0 bis 2 Kohlenstoffatome besitzen.

7. 2-(2′-Difluormethoxy-4′-chlorphenyl)-2-(1H-1,2,4-triazolylmethyl)-4-ethyl-1,3-dioxolan,
2-(4′-Difluormethoxy-2′-tolyl)-2-(1H-1,2,4-triazolylmethyl)-4-ethyl-1,3-dioxolan, oder
2-(4′-Difluormethoxy-2′tolyl)-2-(1H-1,2,4-triazolylmethyl)-4,5-dimethyl-dioxolan gemäss Anspruch 1.

8. Verfahren zur Herstellung von Verbindungen der Formel I nach Anspruch 1, dadurch gekennzeichnet, dass man
A) ein Triazol der Formel II

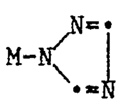
(II),

worin M Wasserstoff oder ein Metallkation darstellt, mit einer Verbindung der Formel III

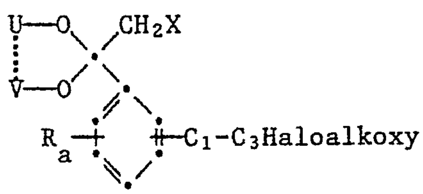
(III),

worin X eine nukleophile Abgangsgruppe bedeutet, kondensiert oder indem man
B) in einer Verbindung der Formel IV

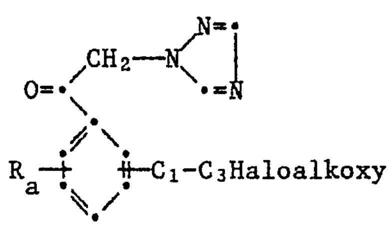
(IV)

die Carbonylfunktion in eine Ketalfunktion der Formel

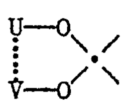
(V)

überführt, oder indem man
C) zur Herstellung von Verbindungen der Formel I, worin U und V gemeinsam eine Gruppe der Formel $-CH_2-CH(CH_2ZR_6')$- darstellen und $R_6'$ einen von Wasserstoff verschiedenen Rest $R_6$ bedeutet,

Verbindungen der Formeln VI und VII miteinander kondensiert,

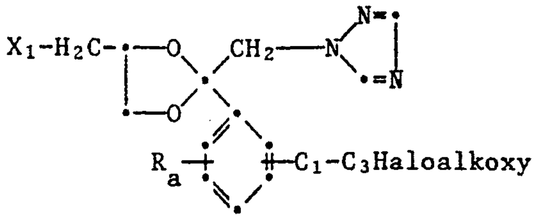

und $R_6-X_2$ (VII)

(VI)

worin einer der Reste $X_1$ und $X_2$ gegebenenfalls in Salzform vorliegendes Hydroxy oder Mercapto, z.B. der Formel -Z-M, und der andere eine nukleophile Abgangsgruppe X bedeutet oder sowohl $X_1$ als auch $X_2$ Hydroxygruppen darstellen; oder indem man

D) Hydrazine der Formel IX,

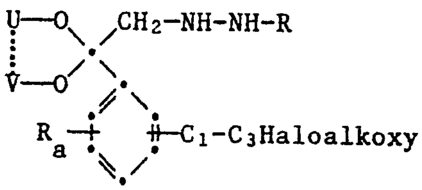

(IX),

a) mit R = -CHO, -COR', -COOR' oder $-CONH_2$ und R'$C_1$-$C_4$-Alkyl, Benzyl oder Phenyl, hydrolysiert und die entstandene Verbindung der Formel IX mit R = H oder ihre Salze mit anorganischen oder organischen Säuren mit Hilfe von Formamid und/oder [3-(Dimethylamino)-2-azaprop-2-en-1-yliden]-dimethylammoniumchlorid (Azasalz), $[(CH_3)_2N \overset{+}{=} CH-N=CH-N(CH_3)_2]Cl^-$), in die Verbindung der Formel I überführt, oder

b) Verbindungen der Formel IX mit R = -COR' mit wässriger Ameisensäure in die N,N'-Bisformylderivate überführt und diese mit Formamid, gegebenenfalls in Gegenwart von $NH_3$ oder einer $NH_3$ liefernden Substanz zu einem Triazol-Derivat der Formel I cyclisiert, wobei R' $C_1$-$C_4$- Alkyl, Benzyl oder Phenyl bedeuten, und, falls erwünscht, eine verfahrensgemäss erhaltene Verbindung in eine andere Verbin dung der Formel I umwandelt und/oder eine verfahrensgemäss erhaltene freie Verbindung in ein Säureadditionssalz, oder ein verfahrensgemäss erhaltenes Säureadditionssalz in die freie Verbindung oder in ein anderes Säureadditionssalz, oder eine verfahrensgemäss erhaltene Verbindung bzw. ein verfahrensgemäss erhaltenes Säureadditionssalz in einen Metallkomplex überführt; wobei die Substituenten in den obigen Formeln die unter Formel I angegebenen Bedeutungen haben.

9. Schädlingsbekämpfungsmittel zur Bekämpfung oder Verhütung eines Befalls durch Mikroorganismen, dadurch gekennzeichnet, dass es als mindestens eine aktive Komponente eine Verbindung der Formel I gemäss Anspruch 1 enthält.

10. Mittel gemäss Anspruch 9, dadurch gekennzeichnet, dass es als mindestens eine aktive Komponente eine Verbindung der Formel I gemäss Anspruch 2 enthält.

11. Mittel gemäss Anspruch 9, dadurch gekennzeichnet, dass es als mindestens eine aktive Komponente eine Verbindung der Formel I gemäss Anspruch 3 enthält.

12. Mittel gemäss Anspruch 9, dadurch gekennzeichnet, dass es als mindestens eine aktive Komponente eine Verbindung der Formel I gemäss Anspruch 4 enthält.

13. Mittel gemäss Anspruch 9, dadurch gekennzeichnet, dass es als mindestens eine aktive Komponente eine Verbindung der Formel I gemäss Anspruch 5 enthält.

14. Mittel gemäss Anspruch 9, dadurch gekennzeichnet, dass es als mindestens eine aktive Komponente eine Verbindung der Formel I gemäss Anspruch 6 enthält.

15. Mittel gemäss Anspruch 9, dadurch gekennzeichnet, dass es als mindestens eine aktive Komponente eine Verbindung gemäss Anspruch 7 enthält.

16. Mittel nach Anspruch 9, dadurch gekennzeichnet, dass es 0,2 bis 99 % eines Wirkstoffs der Formel I, 99,9 bis 1 % eines festen oder flüssigen Zusatzstoffes und 0 bis 25 % eines Tensides enthält.

17. Verfahren zur Bekämpfung oder Verhütung eines Befalls von Kulturpflanzen durch phytopathogene Mikroorganismen, dadurch gekennzeichnet, dass man eine Verbindung der Formel I gemäss Anspruch 1 auf die Pflanze oder ihren Standort oder auf Pflanzenteile appliziert.

18. Verfahren gemäss Anspruch 17, dadurch gekennzeichnet, dass die Pflanzenteile das Saatgut sind.

19. Verfahren gemäss Anspruch 17, dadurch gekennzeichnet, dass Reispflanzen behandelt werden.

20. Verfahren gemäss Anspruch 19, dadurch gekennzeichnet, dass Reispflanzen mit dem Wirkstoff 2-(2'-Difluormethoxy-4'-chlorphenyl)-2-(1H-1,2,4-triazolylmethyl)-4-ethyl-1,3-dioxolan behandelt werden.

21. Verbindungen der Formel III

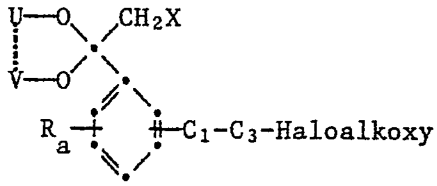

(III)

in welcher einer der beiden Phenylsubstituenten in 2-Stellung und der andere in 4-Stellung steht, und worin

$R_a$ Halogen, Methyl oder $C_1$-$C_3$-Haloalkoxy bedeutet, und worin

U und V unabhängig voneinander gegebenenfalls durch Halogen oder $C_1$-$C_6$-Alkoxy substituiertes $C_1$-$C_{12}$-Alkyl darstellen oder zusammen eine der folgenden Alkylenbrücken

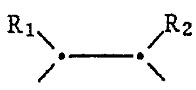

oder

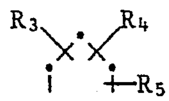

bilden, wobei

$R_1$ und $R_2$ unabhängig voneinander für Wasserstoff, $C_1$-$C_6$-Alkyl, ein- oder mehrfach durch Halogen substituiertes $C_1$-$C_6$-Alkyl, Phenyl, ein- oder mehrfach durch Halogen und/oder $C_1$-$C_2$-Alkyl substituiertes Phenyl oder die Gruppe $-CH_2$-Z-$R_6$ stehen oder worin $R_1$ und $R_2$ zusammen eine unsubstituierte oder durch $C_1$-$C_4$-Alkyl substituierte Tetramethylenbrücke bilden; und wobei

Z Sauerstoff oder Schwefel bedeutet;

$R_6$ für Wasserstoff, $C_1$-$C_6$-Alkyl, ein- oder mehrfach durch Halogen oder $C_1$-$C_3$-Alkoxy substituiertes $C_1$-$C_6$-Alkyl, oder für $C_3$-$C_4$-Alkenyl, 2-Propinyl (=Propargyl), 3-Halo-2-propinyl, Phenyl, oder Benzyl, deren aromatischer Ring unsubstituiert oder durch Halogen, $C_1$-$C_3$-Alkyl, $C_1$-$C_3$-Alkoxy, Nitro und/oder $CF_3$ ein-oder mehrfach substituiert ist, steht;

$R_3$, $R_4$ und $R_5$ unabhängig voneinander Wasserstoff oder $C_1$-$C_4$-Alkyl bedeuten, wobei die Gesamtzahl der Kohlenstoffatome in $R_3$, $R_4$ und $R_5$ die Zahl 6 nicht übersteigt;

und worin X eine nukleophile Abgangsgruppe bedeutet.

22. Verbindungen der Formel III gemäss Anspruch 21 worin X Halogen, Benzolsulfonyloxy, p-Tosyloxy, Trifluoracetyloxy oder Niederalkylsulfonyloxy bedeutet.